# EUROPEAN PATENT SPECIFICATION

(11) **EP 2 637 665 B1**
(45) Date of publication and mention of the grant of the patent: **08.04.2015**
(21) Application number: 11791122.2
(22) Date of filing: 11.11.2011
(51) Int. Cl.: A61K 31/5377, A61K 31/573, A61P 35/00

(54) **DEXAMETHASONE COMBINATION THERAPY**
DEXAMETHASON-KOMBINATIONSTHERAPIE
POLYTHÉRAPIE À LA DEXAMÉTHASONE

(30) Priority: 12.11.2010 US 413260 P; 06.12.2010 US 420089 P
(43) Date of publication of application: 18.09.2013
(73) Proprietor: Board of Regents, The University of Texas System, Austin, TX 78701 (US)
(72) Inventor: YI, Qing, Pearland, TX 77584 (US); ZHENG, Yuhuan, Houston, TX 77054 (US)
(74) Representative: Clyde-Watson, Zöe
(86) International application number: PCT/US2011/060297
(87) International publication number: WO 2012/065021

(56) References cited:
- WO-A1-2007/084786
- DATABASE BIOSIS [Online] BIOSCIENCES INFORMATION SERVICE, PHILADELPHIA, PA, US; November 2009 (2009-11), WALSH KATHERINE J ET AL: "PI3K Inhibitors Inhibit Lymphoma Growth by Downregulation of MYC-Dependent Proliferation.", XP002666170, Database accession no. PREV201000352691 & BLOOD, vol. 114, no. 22, November 2009 (2009-11), page 676, 51ST ANNUAL MEETING OF THE AMERICAN-SOCIETY-OF-HEMATOLOGY; NEW ORLEANS, LA, USA; DECEMBER 05 -08, 2009 ISSN: 0006-4971(print)
- DATABASE HCAPLUS [Online] 13 August 2010 (2010-08-13), BURGER, MATTHEW T. ET AL: "Discovery of BKM120 , a pan class I PI3 kinase inhibitor in phase I/II clinical trials", XP002666171, Database accession no. 2010:1012092 & ABSTRACTS OF PAPERS, 240TH ACS NATIONAL MEETING, BOSTON, MA, UNITED STATES, 22 August 2010 (2010-08-22), - 26 August 2010 (2010-08-26), MEDI-489 PUBLISHER: AMERICAN CHEMICAL SOCIETY, WASHINGTON, D. C.

## Description

### Related Applications

This application claims priority to U.S. Provisional Application No. 61/413,260, Attorney Docket No. NVT-183-1, filed November 12,2010, titled "COMBINATION THERAPY." This application also claims priority to U.S. Provisional Application No. 61/420,089, Attorney Docket No. NVT-183-2, filed December 6,2010, titled "COMBINATION THERAPY."

### Background

Phosphatidylinositol 3-kinase (PI3K) plays a central role in cell metabolism. PI3K is activated by growth factors, cytokines, and other stimulatory factors in association with their receptors. Activated PI3K in turn initiates signaling transduction to Akt-mTOR and leads to regulation of cell growth, proliferation, and apoptosis. Dysregulation of the pathway is widely seen in different types of human cancers, including multiple myeloma (MM). Therefore, PI3K-Akt inhibition is expected to exert broad anti-MM activity. The compound 5-(2,6-di-moipholin-4-yl-pyrimidin-4-yl)-4-trifluoromethyl-pyridin-2-ylamine (Compound A) is a pan-PI3K inhibitor. This compound has shown significant cell growth inhibition and induction of apoptosis in a variety of tumor cell lines. Compound A is currently being investigated in Phase I clinical trials in solid tumor patients.

Multiple myeloma (MM) is a malignant B-cell tumor characterized by proliferation of plasma cells in the bone marrow (Kyle RA, Rajkumar SV. Multiple myeloma. N Engl JMed. 2004;351 (18):1860-1873). MM is accompanied by lytic bone lesions, high-level production of monoclonal immunoglobulin (Ig), and suppression of normal Ig production and hematopoiesis (Dvorak C. Common complaints, difficult diagnosis: multiple myeloma. J Am Acad Nurse Pract. 2006;18 (5):190-194). Chemotherapy is the most conventional treatment for MM patients (Jagannath S, Kyle RA, Palumbo A, Siegel DS, Cunningham S, Berenson J. The current status and future of multiple myeloma in the clinic. Clin Lymphoma Myeloma Leuk;10 (1):28-43). However, despite the improvement of chemotherapy and introduction of new drugs, MM is still an incurable disease. In the United States, MM accounts for nearly 10% of deaths caused by hematological malignancies (Jemal A, Siegel R, Ward E, Hao Y, Xu J, Thun MJ. Cancer statistics, 2009. CA Cancer J Clin. 2009;59 (4):225-249). Therefore, the development of new chemotherapy agents is an ongoing effort in MM research.

### Summary of the Invention

There remains a need for new chemotherapeutic treatments for multiple myeloma.

Thus, in one aspect, provided herein is a combination for use in the treatment of multiple myeloma said combination comprising (1) 5-(2,6-di-morpholin-4-yl-pyrimidin-4-yl)-4-trifluoromethyl- pyridin-2-ylamine (i.e. compound A), and/or a stereoisomer, tautomer, or pharmaceutically acceptable salt thereof, and (2) dexamethasone and/or a pharmaceutically acceptable salt, solvate, or racemate thereof. In another aspect, provided herein is a combination for use in the treatment of multiple myeloma which combination comprises (1) 5-(2,6-di-morpholin-4-yl-pyrimidin-4-yl)-4-trifluoromethyl- pyridin-2-ylamine (i.e. compound A) and (2) dexamethasone.

In one embodiment of the uses, the subject is human. In another embodiment, (1) and (2) are co-administered. In yet another embodiment, (1) and' (2) are in a single formulation or unit dosage form. In still another embodiment, (1) and (2) are in separate formulations or unit dosage forms.

In another embodiment, (1) and (2) are administered at substantially the same time. In yet another embodiment, (1) and (2) are administered at different times. In still another embodiment, (1) and/or (2) is administered at dosages that would not be effective when one or both of (1) and (2) is administered alone, but which are effective in combination.

In another aspect, provided herein is a pharmaceutical formulation comprising an amount of (1) 5-(2,6-di-morpholin-4-yl-pyrimidin-4-yl)-4-trifluoromethyl- pyridin-2-ylamine (i.e. compound A), and/or a stereoisomer, tautomer, or pharmaceutically acceptable salt thereof, and an amount of (2) dexamethasone and/or a pharmaceutically acceptable salt, solvate, or racemate thereof, wherein the combined amount of (1) and (2) is effective for treatment of multiple myeloma. In yet another aspect, provided herein is a pharmaceutical formulation comprising an amount of (1) compound A, and an amount of (2) dexamethasone, wherein the combined amount of (1) and (2) is effective for treatment of multiple myeloma.

In one embodiment of the pharmaceutical formulations, the amount of (1) and the amount of (2) are in a single formulation or unit dosage form. In another embodiment, the formulation or unit dosage form is an oral formulation or unit dosage form. In yet another embodiment, the amount of (1) and/or the amount of (2) would not be effective when one or both of (1) and (2) is administered alone, but which amounts are effective in combination.

In another aspect, provided herein is a composition comprising (1) compound A and (2) dexamethasone. In still another aspect, provided herein is a combination comprising compound A and dexamethasone for the treatment of multiple myeloma.

In another aspect, provided herein is a, combination for use in the treatment of multiple myeloma said combination comprising (1) 5-(2,6-di-morpholin-4-yl-pyrimidin-4-yl)-trifluoromethyl-pyridin-2-ylamine, and/or a stereoisomer, tautomer, or pharmaceutically acceptable salt thereof, and (2) dexamethasone and/or a pharmaceutically acceptable salt, solvate, or racemate thereof. In yet another aspect, provided herein is a combination for use in the treatment of multiple myeloma which combination comprises (1) 5-(2,6-di-morpholin-4-yl-pyrimidin-4-yl)-4-trifluoromethyl-pyridin-2-ylamine and (2) dexamethasone.

In one embodiment of the uses, the subject is human. In another embodiment, (1) and (2), are co-administered.

In yet another embodiment, (1) and (2) are in a single formulation or unit dosage form. In still another embodiment, (1) and ' (2) are in a separate formulations or unit dosage forms. In another embodiment (1) and (2) are administered at substantially the same time. In yet another embodiment, (1) and (2) are administered at different times. In still another embodiment, (1) and/or (2) is administered at dosages that would not be effective when one or both of (1) and (2) is administered alone, but which are effective in combination.

In another aspect, provided herein is a pharmaceutical formulation comprising an amount of (1) 5-(2,6-di-morpholin-4-yl-pyrimidin-4-yl)-4-trifluoromethyl-pyridin-2-ylamine, and/or a stereoisomer, tautomer, or pharmaceutically acceptable salt thereof, and an amount of (2) dexamethasone and/or a pharmaceutically acceptable salt, solvate, or racemate thereof, wherein the combined amount of (1) and (2) is effective for treatment of multiple myeloma.

In yet another aspect, provided herein is a pharmaceutical formulation comprising an amount of (1) 5-(2,6-di-morpholin-4-yl-pyrimidin-4-yl)-4-trifluoromethyl-pyridin-2-ylamine, and an amount of (2) dexamethasone, wherein the combined amount of (1) and (2) is effective for treatment of multiple myeloma.

In one embodiment of the pharmaceutical formulations, the amount of (1) and the amount of (2) are in a single formulation or unit dosage form. In another embodiment, the formulation or unit dosage form is an oral formulation or unit dosage form. In yet another embodiment, the amount of (1) and/or the amount of (2) would not be effective when one or both of (1) and (2) is administered alone, but which amounts are effective in combination.

In another aspect, provided herein is a composition comprising 5-(2,6-di-morpholin-4-yl-pyrimidin-4-yl)-4-trifluoromethyl-pyridin-2-ylamine, and dexamethasone.

In still another aspect, provided herein is a combination comprising 5-(2,6-di-morpholin-4-yl-pyrimidin-4-yl)-4-trifluoromethyl-pyridin-2-ylamine, and dexamethasone for the treatment of multiple myeloma.

### Brief Description of Drawings

**Figures 1A-1E** show that compound A induces MM cells growth repression and apoptosis, but has limited cytotoxicity to normal PBMCs.
**Figures 2A-2B** show that the presence of BMSCs or IL-6 does not attenuate compound A induced MM cell apoptosis.
**Figures 3A-3B** show that compound A treatment causes cell cycle arrest at G1 phase.
**Figures 4A-4C** show that compound A induced intracellular signaling and caspases activation in MM cells.
**Figures 5A-5F** show that compound A and dexamethasone have synergistic anti-MM activity.
**Figures 6A-6C** demonstrate *in vivo* therapeutic effects of compound A on established MM model in SCID mice.

### Detailed Description

It has been discovered that administering a combination of compound A and dexamethasone provides surprising, synergistic effects for treating multiple myeloma in a subject. Stereoisomers, tautomers, or pharmaceutically acceptable salts of compound A and pharmaceutically acceptable salts, solvates, or racemates of dexamethasone also can be used in the combination therapies disclosed herein. Such an approach - combination or coadministration of the two types of agents - is particularly useful for treating individuals suffering from multiple myeloma who do not respond to or are resistant to currently-available therapies and is also useful for improving the efficacy and/or reducing the side effects of currently-available multiple myeloma therapies for individuals who do respond to such therapies.

Certain terms used herein are described below. Compounds of the present invention are described using standard nomenclature. Unless defined otherwise, all technical and scientific terms used herein have the same meaning as is commonly understood by one of skill in the art to which this invention belongs.

Provided herein is a combination of therapeutic agents and the combination of agents for use in the treatment of multiple myeloma. As used herein, a "combination of agents" and similar terms refer to a combination of two types of agents: (1) compound A and/or a stereoisomer, tautomer, or pharmaceutically acceptable salt thereof, and (2) dexamethasone and/or a pharmacutically acceptable salt thereof. Use of isomeric or racemic mixtures of the individual agents also is provided.

WO07/084786 describes pyrimidine derivatives, which have been found to inhibit the activity of lipid kinases, such as phosphatidylinositol 3-kinase (PI3K). Specific pyrimidine derivatives, their preparation and suitable pharmaceutical formulations containing the same are described in WO07/084786 and include compounds of formula (I): 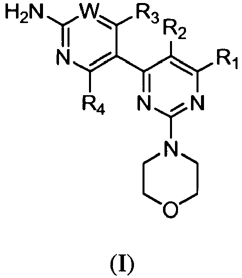 or a stereoisomer, tautomer, or pharmaceutically acceptable salt thereof, wherein W is CR_{w} or N, wherein
R_{w} is selected from the group consisting of:
   (1) hydrogen,
   (2) cyano,
   (3) halogen,
   (4) methyl,
   5) trifluoromethyl,
   (6) sulfonamide;
R₁ is selected from the group consisting of:
   (1) hydrogen,
   (2) cyano,
   (3) nitro,
   (4) halogen,
   (5) substituted and unsubstituted alkyl,
   (6) substituted and unsubstituted alkenyl,
   (7) substituted and unsubstituted alkynyl,
   (8) substituted and unsubstituted aryl,
   (9) substituted and unsubstituted heteroaryl,
   (10) substituted and unsubstituted heterocyclyl,
   (11) substituted and unsubstituted cycloalkyl,
   (12) -COR₁ₐ,
   (13) -CO₂R₁ₐ,
   (14) -CONR₁ₐR_{1b},
   (15) -NR₁ₐR_{1b},
   (16) -NR₁ₐCOR_{1b},
   (17) -NR₁ₐSO₂R_{1b},
   (18) -OCOR₁ₐ,
   (19) -OR₁ₐ,
   (20) -SR₁ₐ,
   (21) -SOR₁ₐ,
   (23) -SO₂NR₁ₐR_{1b} wherein
R₁ₐ, and R_{1b} are independently selected from the group consisting of:
   (a) hydrogen,
   (b) substituted or unsubstituted alkyl,
   (c) substituted and unsubstituted aryl,
   (d) substituted and unsubstituted heteroaryl,
   (e) substituted and unsubstituted heterocyclyl, and
   (f) substituted and unsubstituted cycloalkyl;
R₂ is selected from the group consisting of:
   (1) hydrogen,
   (2) cyano,
   (3) nitro,
   (4) halogen,
   (5) hydroxy,
   (6) amino,
   (7) substituted and unsubstituted alkyl,
   (8) -COR₂ₐ, and
   (9) -NR₂ₐCOR_{2b}, wherein
R₂ₐ, and R_{2b} are independently selected from the group consisting of:
   (a) hydrogen, and
   (b) substituted or unsubstituted alkyl;
R₃ is selected from the group consisting of:
   (1) hydrogen,
   (2) cyano,
   (3) nitro,
   (4) halogen,
   (5) substituted and unsubstituted alkyl,
   (6) substituted and unsubstituted alkenyl,
   (7) substituted and unsubstituted alkynyl,
   (8) substituted and unsubstituted aryl,
   (9) substituted and unsubstituted heteroaryl,
   (10) substituted and unsubstituted heterocyclyl,
   (11) substituted and unsubstituted cycloalkyl,
   (12) -COR₃ₐ,
   (14) -NR₃ₐR_{3b}
   (13) -NR₃ₐCOR_{3b},
   (15) -NR₃ₐSO₂R_{3b},
   (16) -OR₃ₐ,
   (17) -SR₃ₐ,
   (18) -SOR₃ₐ,
   (19) -SO₂R₃ₐ, wherein
R₃ₐ, and R_{3b} are independently selected from the group consisting of:
   (a) hydrogen,
   (b) substituted or unsubstituted alkyl,
   (c) substituted and unsubstituted aryl,
   (d) substituted and unsubstituted heteroaryl,
   (e) substituted and unsubstituted heterocyclyl, and
   (f) substituted and unsubstituted cycloalkyl; and
R₄ is selected from the group consisting of
   (1) hydrogen, and
   (2) halogen.

As used herein, the term "alkyl" refers to alkyl groups that do not contain heteroatoms. Thus the phrase includes straight chain alkyl groups such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example:-CH(CH₃)₂, -CH(CH₃)(CH₂CH₃), -CH(CH₂CH₃)₂, -C(CH₃)₃, -C(CH₂CH₃)₃,-CH₂CH(CH₃)₂, -CH₂CH(CH₃)(CH₂CH₃), -CH₂CH(CH₂CH₃)₂, -CH₂C(CH₃)₃, -CH₂C(CH ₂CH₃)₃, -CH(CH₃)-CH(CH₃)(CH₂CH₃), -CH₂CH₂CH(CH₃)₂, -CH₂CH₂CH(CH₃)(CH₂CH₃), -CH₂CH₂CH(CH₂CH₃)₂, -CH₂CH₂C(CH₃)₃, -CH₂CH₂C(CH₂CH₃)₃, -CH(CH₃)CH₂-CH(CH₃)₂, -CH(CH₃)CH(CH₃)CH(CH₃)₂, -CH(CH₂CH₃)CH(CH₃)CH(CH₃)(CH₂CH₃), and others. Thus the phrase "alkyl groups" includes primary alkyl groups, secondary alkyl groups, and tertiary alkyl groups. Preferred alkyl groups include straight and branched chain alkyl groups having 1 to 12 carbon atoms or 1 to 6 carbon atoms.

The term "alkenyl," refers to straight chain, branched, or cyclic groups from 2 to about 20 carbon atoms such as those described with respect to alkyl groups as defined above, except having one or more carbon-carbon double bonds. Examples include, but are not limited to vinyl, -CH=C(H)(CH₃), -CH=C(CH₃)₂, -C(CH₃)=C(H)₂,-C(CH₃)=C(H)(CH₃), -C(CH₂CH₃)=CH₂, cyclohexenyl, cyclopentenyl, cyclohexadienyl, butadienyl, pentadienyl, and hexadienyl among others. Preferred alkenyl groups include straight chain and branched alkenyl groups and cyclic alkenyl groups having 2 to 12 carbon atoms or 2 to 6 carbon atoms.

The term "alkynyl" refers to straight chain, branched, or cyclic groups from 2 to about 20 carbon atoms such as those described with respect to alkyl groups as defined above, except having one or more carbon-carbon triple bonds. Examples include, but are not limited to -C≡C(H), -C≡C(CH₃), -C≡C(CH₂CH₃), -C(H₂)C≡C(H),-C(H)₂C≡C(CH₃), and -C(H)₂C≡C(CH₂CH₃) among others. Preferred alkynyl groups include straight chain and branched alkynyl groups having 2 to 12 carbon atoms or 2 to 6 carbon atoms.

Alkyl, alkenyl, and alkynyl groups may be substituted. "Substituted alkyl" refers to an alkyl group as defined above in which one or more bonds to a carbon(s) or hydrogen(s) are replaced by a bond to non-hydrogen and non-carbon atoms such as, but not limited to, a halogen atom such as F, Cl, Br, and I; an oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as in trialkylsilyl groups, dialkylarylsilyl groups, alkyldiarylsilyl groups, and triarylsilyl groups; and other heteroatoms in various other groups. Substituted alkyl groups also include groups in which one or more bonds to a carbon(s) or hydrogen(s) atom is replaced by a higher-order bond (*e.g*., a double- or triple-bond) to a heteroatom such as oxygen in oxo, carbonyl, carboxyl, and ester groups; nitrogen in groups such as imines, oximes, hydrazones, and nitriles. Substituted alkyl groups further include alkyl groups in which one or more bonds to a carbon(s) or hydrogen(s) atoms is replaced by a bond to an aryl, heteroaryl, heterocyclyl, or cycloalkyl group. Preferred substituted alkyl groups include, among others, alkyl groups in which one or more bonds to a carbon or hydrogen atom is/are replaced by one or more bonds to fluoro, chloro, or bromo group. Another preferred substituted alkyl group is the trifluoromethyl group and other alkyl groups that contain the trifluoromethyl group. Other preferred substituted alkyl groups include those in which one or more bonds to a carbon or hydrogen atom is replaced by a bond to an oxygen atom such that the substituted alkyl group contains a hydroxyl, alkoxy, or aryloxy group. Other preferred substituted alkyl groups include alkyl groups that have an amine, or a substituted or unsubstituted alkylamine, dialkylamine, arylamine, (alkyl)(aryl)amine, diarylamine, heterocyclylamine, diheterocyclylamine, (alkyl)(heterocyclyl)amine, or (aryl)(heterocyclyl)amine group. Still other preferred substituted alkyl groups include those in which one or more bonds to a carbon(s) or hydrogen(s) atoms is replaced by a bond to an aryl, heteroaryl, heterocyclyl, or cycloalkyl group. Examples of substituted alkyl are: -(CH₂)₃NH₂,-(CH₂)₃NH(CH₃), -(CH₂)₃NH(CH₃)₂, -CH₂C(=CH₂)CH₂NH₂, -CH₂C(=O)CH₂NH₂,-CH₂S(=O)₂CH₃, -CH₂OCH₂NH₂, -CO₂H. Examples of substituents of substituted alkyl are: -CH₃, -C₂H₅, -CH₂OH, -OH, -OCH₃, -OC₂H₅, -OCF₃, -OC(=O)CH₃,-OC(=O)NH₂, -OC(=O)N(CH₃)₂, -CN, -NO₂, -C(=O)CH₃, -CO₂H, -CO₂CH₃, -CONH₂,-NH₂,-N(CH₃)₂, -NHSO₂CH₃, -NHCOCH₃, -NHC(=O)OCH₃, -NHSO-₂CH₃, -SO₂CH₃, -SO₂NH₂, halo.

The term "substituted alkenyl" has the same meaning with respect to alkenyl groups that substituted alkyl groups had with respect to unsubstituted alkyl groups. A substituted alkenyl group includes alkenyl groups in which a non-carbon or non-hydrogen atom is bonded to a carbon double bonded to another carbon and those in which one of the non-carbon or non-hydrogen atoms is bonded to a carbon not involved in a double bond to another carbon.

The term "substituted alkynyl" has the same meaning with respect to alkynyl groups that substituted alkyl groups had with respect to unsubstituted alkyl groups. A substituted alkynyl group includes alkynyl groups in which a non-carbon or non-hydrogen atom is bonded to a carbon triple bonded to another carbon and those in which a non-carbon or non-hydrogen atom is bonded to a carbon not involved in a triple bond to another carbon.

The term "alkoxy" refers to RO- wherein R is alkyl. Representative examples of alkoxy groups include methoxy, ethoxy, t-butoxy, trifluoromethoxy, and the like.

The term "halogen" or "halo" refers to chloro, bromo, fluoro, and iodo groups. The term "haloalkyl" refers to an alkyl radical substituted with one or more halogen atoms. The term "haloalkoxy" refers to an alkoxy radical substituted with one or more halogen atoms.

The term "alkoxyalkyl" refers to the group -alk₁-O-alk₂ where alk₁ is alkyl or alkenyl, and alk₂ is alkyl or alkenyl. The term "aryloxyalkyl" refers to the group -alkyl O-aryl. The term "aralkoxyalkyl" refers to the group -alkylenyl-O-aralkyl.

The term "carbonyl" refers to the divalent group -C(O)-.

The term "cycloalkyl" refers to a mono- or polycyclic, heterocyclic or carbocyclic alkyl substituent. Representative cycloalkyl groups include cyclopropyl, cyclobutyl, cyclopentyl, cyclohexyl, cycloheptyl, and cyclooctyl and such rings substituted with straight and branched chain alkyl groups as defined above. Typical cycloalkyl substituents have from 3 to 8 backbone (*i.e.,* ring) atoms in which each backbone atom is either carbon or a heteroatom. The term "heterocycloalkyl" refers herein to cycloalkyl substituents that have from 1 to 5, and more typically from 1 to 4 heteroatoms in the ring structure. Suitable heteroatoms employed in compounds of the present invention are nitrogen, oxygen, and sulfur. Representative heterocycloalkyl moieties include, for example, morpholino, piperazinyl, piperadinyl, and the like. Carbocycloalkyl groups are cycloalkyl groups in which all ring atoms are carbon. When used in connection with cycloalkyl substituents, the term "polycyclic" refers herein to fused and non-fused alkyl cyclic structures.

The term "aryl" refers to optionally substituted monocyclic and polycyclic aromatic groups having from 3 to 14 backbone carbon or hetero atoms, and includes both carbocyclic aryl groups and heterocyclic aryl groups. The term refers to, but is not limited to, groups such as phenyl, biphenyl, anthracenyl, naphthenyl by way of example. Carbocyclic aryl groups are aryl groups in which all ring atoms in the aromatic ring are carbon. The term "heteroaryl" refers herein to aryl groups having from 1 to 4 heteroatoms as ring atoms in an aromatic ring with the remainder of the ring atoms being carbon atoms.

The term "unsubstituted aryl" includes groups containing condensed rings such as naphthalene. It does not include aryl groups that have other groups such as alkyl or halo groups bonded to one of the ring members, as aryl groups such as tolyl are considered herein to be substituted aryl groups as described below. A preferred unsubstituted aryl group is phenyl. Unsubstituted aryl groups may be bonded to one or more carbon atom(s), oxygen atom(s), nitrogen atom(s), and/or sulfur atom(s) in the parent compound, however.

The term "substituted aryl group" has the same meaning with respect to unsubstituted aryl groups that substituted alkyl groups had with respect to unsubstituted alkyl groups. However, a substituted aryl group also includes aryl groups in which one of the aromatic carbons is bonded to one of the non-carbon or non-hydrogen atoms described above and also includes aryl groups in which one or more aromatic carbons of the aryl group is bonded to a substituted and/or unsubstituted alkyl, alkenyl, or alkynyl group as defined herein. This includes bonding arrangements in which two carbon atoms of an aryl group are bonded to two atoms of an alkyl, alkenyl, or alkynyl group to define a fused ring system (e.g., dihydronaphthyl or tetrahydronaphthyl). Thus, the phrase "substituted aryl" includes, but is not limited to tolyl, and hydroxyphenyl among others.

The term "substituted heteroaryl" as used herein refers to a heteroaryl group as defined herein substituted by independent replacement of one, two or three of the hydrogen atoms thereon with Cl, Br, F, I, -OH, -CN, C₁-C₃-alkyl, C₁-C₆-alkoxy, C₁-C₆-alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substituent may be an aryl, heteroaryl, or heterocycloalkyl group.

The term "substituted heterocycle," "heterocyclic group," "heterocycle," or "heterocyclyl," as used herein refers to any 3- or 4-membered ring containing a heteroatom selected from nitrogen, oxygen, and sulfur or a 5- or 6-membered ring containing from one to three heteroatoms selected from the group consisting of nitrogen, oxygen, or sulfur; wherein the 5-membered ring has 0-2 double bonds and the 6-membered ring has 0-3 double bonds; wherein the nitrogen and sulfur atom maybe optionally oxidized; wherein the nitrogen and sulfur heteroatoms maybe optionally quarternized; and including any bicyclic group in which any of the above heterocyclic rings is fused to a benzene ring or another 5- or 6-membered heterocyclic ring independently defined above. Examples of heterocyclyl groups include, but are not limited to: unsaturated 3- to 8-membered rings containing 1 to 4 nitrogen atoms such as, but not limited to pyrrolyl, dihydropyridyl, pyrimidyl, pyrazinyl, tetrazolyl, (e.g., 1H-tetrazolyl, 2H-tetrazolyl); condensed unsaturated heterocyclic groups containing 1 to 4 nitrogen atoms such as, but not limited to, isoindolyl, indolinyl, indolizinyl, quinolyl, indazolyl; unsaturated 3- to 8-membered rings containing 1 to 2 oxygen atoms and 1 to 3 nitrogen atoms such as, but not limited to, oxadiazolyl (*e.g.,* 1,2,4-oxadiazolyl, 1,3,4-oxadiazolyl, 1,2,5-oxadiazolyl); saturated 3- to 8-membered rings containing 1 to 2 oxygen atoms and 1 to 3 nitrogen atoms such as, but not limited to, morpholinyl; unsaturated condensed heterocyclic groups containing 1 to 2 oxygen atoms and 1 to 3 nitrogen atoms, for example, benzoxadiazolyl, benzoxazinyl (*e.g.,* 2H-1,4-benzoxazinyl); unsaturated 3- to 8-membered rings containing 1 to 3 sulfur atoms and 1 to 3 nitrogen atoms such as, but not limited to, thiadiazolyl (*e.g.,* 1,2,3-thiadiazolyl, 1,2,4-thiadiazolyl, 1,3,4-thiadiazolyl, 1,2,-thiadiazolyl); saturated 3- to 8-membered rings containing 1 to 2 sulfur atoms and 1 to 3 nitrogen atoms such as, but not limited to, thiazolodinyl; saturated and unsaturated 3- to 8-membered rings containing 1 to 2 sulfur atoms such as, but not limited to, dihydrodithienyl, dihydrodithionyl, tetrahydrothiophene, tetra-hydrothiopyran; unsaturated condensed heterocyclic rings containing 1 to 2 sulfur atoms and 1 to 3 nitrogen atoms such as, but not limited to, benzothiadiazolyl, benzothiazinyl (*e.g.,* 2H-1,4-benzothiazinyl), dihydrobenzothiazinyl (*e.g.,* 2H-3,4-dihydrobenzothiazinyl), unsaturated 3- to 8-membered rings containing oxygen atoms such as, but not limited to furyl; unsaturated condensed heterocyclic rings containing 1 to 2 oxygen atoms such as benzodioxoyl (*e.g.,* 1,3-benzodioxoyl); unsaturated 3- to 8-membered rings containing an oxygen atom and 1 to 2 sulfur atoms such as, but not limited to, dihydrooxathienyl; saturated 3- to 8-membered rings containing 1 to 2 oxygen atoms and 1 to 2 sulfur atoms such as 1,4-oxathiane; unsaturated condensed rings containing 1 to 2 sulfur atoms such as benzodithienyl; and unsaturated condensed heterocyclic rings containing an oxygen atom and 1 to 2 oxygen atoms such as benzoxathienyl. Preferred heterocycles include, for example: diazapinyl, pyrryl, pyrrolinyl, pyrrolidinyl, pyrazolyl, pyrazolinyl, pyrazolidinyl, imidazoyl, imidazolinyl, imidazolidinyl, pyridyl, piperidinyl, pyrazinyl, piperazinyl, N-methyl piperazinyl, azetidinyl, N-methylazetidinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, triazolyl, and benzothienyl. Heterocyclyl groups also include those described above in which one or more S atoms in the ring is double-bonded to one or two oxygen atoms (sulfoxides and sulfones). For example, heterocyclyl groups include tetrahydrothiophene, tetrahydrothiophene oxide, and tetrahydrothiophene 1,1-dioxide. Preferred heterocyclyl groups contain 5 or 6 ring members. More preferred heterocyclyl groups include piperazine, 1,2,3-triazole, 1,2,4-triazole, tetrazole, thiomorpholine, homopiperazine, oxazolidin-2-one, pyrrolidin-2-one, quinuclidine, and tetrahydrofuran.

Heterocyclic moieties can be unsubstituted or monosubstituted or disubstituted with various substituents independently selected from hydroxy, halo, oxo (C=O), alkylimino (RN=, wherein R is alkyl or alkoxy group), amino, alkylamino, dialkylamino, acylaminoalkyl, alkoxy, thioalkoxy, polyalkoxy, alkyl, cycloalkyl or haloalkyl. "Unsubstituted heterocyclyl" includes condensed heterocyclic rings such as benzimidazolyl, it does not include heterocyclyl groups that have other groups such as alkyl or halo groups bonded to one of the ring members as compounds such as 2-methylbenzimidazolyl are substituted heterocyclyl groups.

The heterocyclic groups may be attached at various positions as will be apparent to those having skill in the organic and medicinal chemistry arts in conjunction with the disclosure herein. Non-limiting examples of heterocyclic groups also include the following: 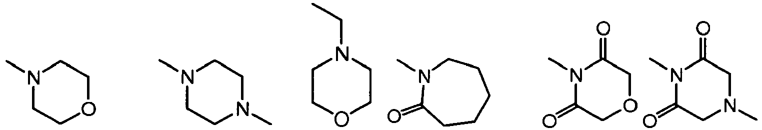 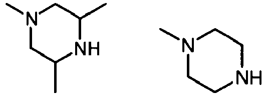 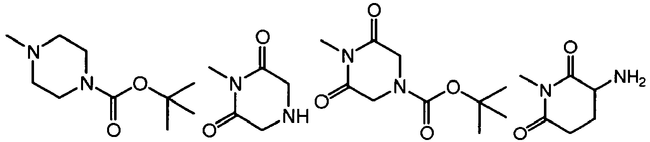 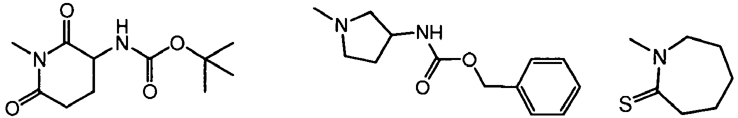 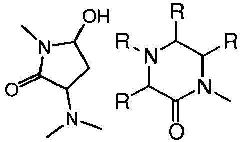 where R is H or a heterocyclic substituent, as described herein.

Representative heterocyclics include, for example, imidazolyl, pyridyl, piperazinyl, azetidinyl, thiazolyl, furanyl, triazolyl benzimidazolyl, benzothiazolyl, benzoxazolyl, quinolinyl, isoquinolinyl, quinazolinyl, quinoxalinyl, phthalazinyl, indolyl, naphthpyridinyl, indazolyl, and quinolizinyl.

The term "optionally substituted" or "substituted" refers to the replacement of hydrogen with one or more monovalent or divalent radical. Suitable substitution groups include, for example, hydroxyl, nitro, amino, imino, cyano, halo, thio, sulfonyl, thioamido, amidino, imidino, oxo, oxamidino, methoxamidino, imidino, guanidino, sulfonamido, carboxyl, formyl, alkyl, substituted alkyl, haloalkyl, alkyamino, haloalkylamino, alkoxy, haloalkoxy, alkoxy-alkyl, alkylcarbonyl, aminocarbonyl, arylcarbonyl, aralkylcarbonyl, heteroarylcarbonyl, heteroaralkyl-carbonyl, alkylthio, aminoalkyl, cyanoalkyl, aryl, benzyl, pyridyl, pyrazolyl, pyrrole, thiophene, imidazolyl, and the like.

The substitution group can itself be substituted. The group substituted onto the substitution group can be carboxyl, halo, nitro, amino, cyano, hydroxyl, alkyl, alkoxy, aminocarbonyl, -SR, thioamido, -SO₃H, -SO₂R, or cycloalkyl, where R is typically hydrogen, hydroxyl or alkyl.

When the substituted substituent includes a straight chain group, the substitution can occur either within the chain (e.g., 2-hydroxypropyl, 2-aminobutyl, and the like) or at the chain terminus (*e.g.,* 2-hydroxyethyl, 3-cyanopropyl, and the like). Substituted substituents can be straight chain, branched or cyclic arrangements of covalently bonded carbon or heteroatoms.

Representative substituted aminocarbonyl groups include, for example, those shown below. These can be further substituted by heterocyclyl groups and heteroaryl groups as will be apparent to those having skill in the organic and medicinal chemistry arts in conjunction with the disclosure herein. Preferred aminocarbonyl groups include: N-(2-cyanoethyl)carboxamide, N-(3-methoxypropyl)carboxamide, N-cyclopropylcarboxamide, N-(2-hydroxy-isopropyl)carboxamide, methyl 2-carbonylamino-3-hydroxypropanoate, N-(2-hydroxypropyl)carboxamide, N-(2-hydroxy-isopropyl)carboxamide, N-[2-hydroxy-1-(hydroxymethyl)ethyl]carboxamide, N-(2-carbonylaminoethyl)acetamide, N-(2-(2-pyridyl)ethyl)carboxamide, N-(2-pyridylmethyl)carboxamide, N-(oxolan-2-ylmethyl)-carboxamide, N-(4-hydroxypyrrolidin-2-yl)carboxamide, N-[2-(2-hydroxyethoxy)ethyl]-carboxamide, N-(4-hydroxycyclohexyl)carboxamide, N-[2-(2-oxo-4-imidazolinyl)ethyl]carboxamide, N-(carbonylaminomethyl)acetamide, N-(3-pyrrolidinylpropyl)carboxamide, N-[1-(carbonylaminomethyl)pyrrolidin-3-yl]acetamide, N-(2-morpholin-4-ylethyl)carboxamide, N-[3-(2-oxopyrrolidinyl)propyl]carboxamide, 4-methyl-2-oxopiperazinecarbaldehyde, N-(2-hydroxy-3-pyrrolidinylpropyl)carboxamide, N-(2-hydroxy-3-morpholin-4-ylpropyl)carboxamide, N-{2-[(5-cyano-2-pyridyl)amino]ethyl}carboxamide, 3-(dimethyl-amino)pyrrolidinecarbaldehyde, N-[(5-methylpyrazin-2-yl)methyl]carboxamide, 2,2,2-trifluoro-N-(1-formylpyrrolidin-3-yl)acetamide,

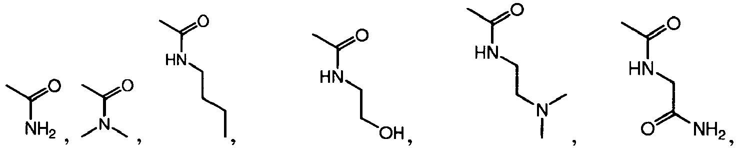

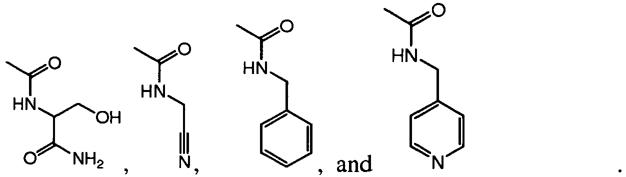

Representative substituted alkoxycarbonyl groups include, for example, those shown below. These alkoxycarbonyl groups can be further substituted as will be apparent to those having skill in the organic and medicinal chemistry arts in conjunction with the disclosure herein.

Representative substituted alkoxycarbonyl groups include, for example, those shown below. These alkoxycarbonyl groups can be further substituted as will be apparent to those having skill in the organic and medicinal chemistry arts in conjunction with the disclosure herein.

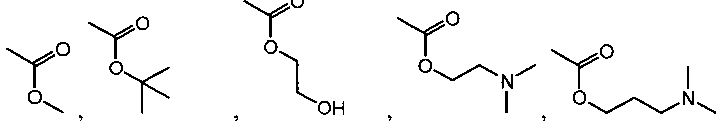

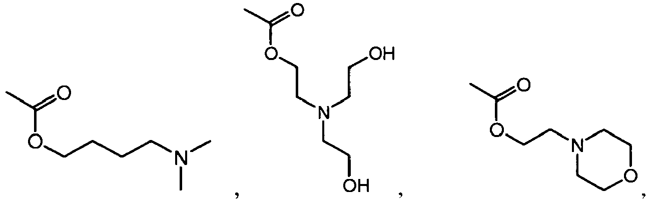

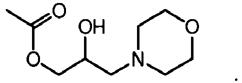

The term "amino" refers herein to the group -NH₂. The term "alkylamino" refers herein to the group -NRR' where R is alkyl and R' is hydrogen or alkyl. The term "arylamino" refers herein to the group -NRR' where R is aryl and R' is hydrogen, alkyl, or aryl. The term "aralkylamino" refers herein to the group -NRR' where R is aralkyl and R' is hydrogen, alkyl, aryl, or aralkyl.

The term "alkoxyalkylamino" refers herein to the group -NR-(alkoxyalkyl), where R is typically hydrogen, aralkyl, or alkyl.

The term "aminocarbonyl" refers herein to the group -C(O)-NH₂. "Substituted aminocarbonyl" refers herein to the group -C(O)-NRR' where R is alkyl and R' is hydrogen or alkyl. The term "arylaminocarbonyl" refers herein to the group -C(O)-NRR' where R is aryl and R' is hydrogen, alkyl or aryl. "Aralkylaminocarbonyl" refers herein to the group -C(O)-NRR' where R is aralkyl and R' is hydrogen, alkyl, aryl, or aralkyl.

The term "amidino" refers to the moieties R-C(=N)-NR'- (the radical being at the "N¹" nitrogen) and R(NR')C=N- (the radical being at the "N²" nitrogen), where R and R' can be hydrogen, alkyl, aryl, or aralkyl.

One compound of formula (I) is the pan-phosphatidylinositol 3-kinase (PI3K) inhibitor 5-(2,6-di-morpholin-4-yl-pyrimidin-4-yl)-4-trifluoromethyl-pyridin-2-ylamine (hereinafter "compound A").

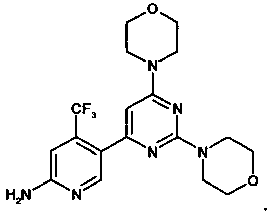

The synthesis of compound A is described in Example 10 of WO 2007/084786.

PI3K plays a central role in cell metabolism (Sasaki T, Takasuga S, Sasaki J, et al. Mammalian phosphoinositide kinases and phosphatases. Prog Lipid Res. 2009;48 (6):307-343; Di Paolo G, De Camilli P. Phosphoinositides in cell regulation and membrane dynamics. Nature. 2006;443 (7112):651-657). PI3K is activated by growth factors, cytokines, and other stimulatory factors in association with their receptors. Activated PI3K in turn initiates signaling transduction to Akt-mTOR and leads to regulation of cell growth, proliferation, and apoptosis (Engelman JA, Luo J, Cantley LC. The evolution of phosphatidylinositol 3-kinases as regulators of growth and metabolism. Nat Rev Genet. 2006;7 (8):606-619). Dysregulation of the pathway is widely seen in different types of human cancers (Bunney TD, Katan M. Phosphoinositide signalling in cancer: beyond PI3K and PTEN. Nat Rev Cancer;10 (5):342-352; Engelman JA. Targeting PI3K signalling in cancer: opportunities, challenges and limitations. Nat Rev Cancer. 2009;9 (8):550-562). In particular in multiple myeloma (MM), a number of myeloma growth factors, such as insulin-like growth factor-1 (IGF-1) and interleukin-6 (IL6), activate PI3K-Akt pathway upon interaction with their receptors on MM cells, and promote MM proliferation, survival and drug resistance (Harvey RD, Lonial S. PI3 kinase/AKT pathway as a therapeutic target in multiple myeloma. Future Oncol. 2007;3 (6):639-647; Younes H, Leleu X, Hatjiharissi E, et al. Targeting the phosphatidylinositol 3-kinase pathway in multiple myeloma. Clin Cancer Res. 2007;13 (13):3771-3775; Hideshima T, Nakamura N, Chauhan D, Anderson KC. Biologic sequelae of interleukin-6 induced PI3-K/Akt signaling in multiple myeloma. Oncogene. 2001;20 (42):5991-6000; Descamps G, Pellat-Deceunynck C, Szpak Y, Bataille R, Robillard N, Amiot M. The magnitude of Akt/phosphatidylinositol 3'-kinase proliferating signaling is related to CD45 expression in human myeloma cells. J Immunol. 2004;173 (8):4953-4959). Therefore, PI3K-Akt inhibition is expected to exert broad anti-MM activity, and several PI3K-Akt inhibitory compounds are under pre-clinical investigation or Phase I and II trials (Harvey RD, Lonial S. PI3 kinase/AKT pathway as a therapeutic target in multiple myeloma. Future Oncol. 2007;3 (6):639-647). Compound A has shown significant cell growth inhibition and induction of apoptosis in a variety of tumor cell lines, and is currently being investigated in Phase I clinical trials in solid tumor patients.

Dexamethasone is designated (8*S,* 9*R*,10*S*,11*S,*13*S,*14*S*,16*R*,17*R*)-9- Fluoro-11,17-dihydroxy-17-(2-hydroxyacetyl)-10,13,16- trimethyl-6,7,8,9,10,11,12,13,14,15,16,17-dodecahydro-3*H*-cyclopenta[a]phenanthren-3-one. It is a potent synthetic glucocorticoid steroid drug, having anti-inflammatory and immunosuppressive activity. Dexamethasone is used to treat certain inflammatory and autoimmune conditions, such as rheumatoid arthritis. It is also used to counteract certain side-effects resulting from antitumor treatment, and is also used as a chemotherapeutic agent in certain hematological malignancies. See, *e.g., Martindale: The Complete Drug Reference,* 37th Edition, published by Pharmaceutical Press.

Administration of the combination of of compound A and dexamethasone includes administration of the combination in a single formulation or unit dosage form, administration of the individual agents of the combination concurrently but separately, or administration of the individual agents of the combination sequentially by any suitable route. The dosage of the individual agents of the combination may require more frequent administration of one of the agent(s) as compared to the other agent(s) in the combination. Therefore, to permit appropriate dosing, packaged pharmaceutical products may contain one or more dosage forms that contain the combination of agents, and one or more dosage forms that contain one of the combination of agents, but not the other agent(s) of the combination.

Agents may contain one or more asymmetric elements such as stereogenic centers or stereogenic axes, *e.g.,* asymmetric carbon atoms, so that the compounds can exist in different stereoisomeric forms. These compounds can be, for example, racemates or optically active forms. For compounds with two or more asymmetric elements, these compounds can additionally be mixtures of diastereomers. For compounds having asymmetric centers, it should be understood that all of the optical isomers and mixtures thereof are encompassed. In addition, compounds with carbon-carbon double bonds may occur in Z- and E-forms; all isomeric forms of the compounds are included in the present invention. In these situations, the single enantiomers (optically active forms) can be obtained by asymmetric synthesis, synthesis from optically pure precursors, or by resolution of the racemates. Resolution of the racemates can also be accomplished, for example, by conventional methods such as crystallization in the presence of a resolving agent, or chromatography, using, for example a chiral HPLC column.

Unless otherwise specified, or clearly indicated by the text, reference to compounds useful in the combination therapy of the invention includes both the free base of the compounds, and all pharmaceutically acceptable salts of the compounds.

As used herein, the term "pharmaceutically acceptable salts" refers to the nontoxic acid or alkaline earth metal salts of the pyrimidine compounds of the invention. These salts can be prepared *in situ* during the final isolation and purification of the pyrimidine compounds, or by separately reacting the base or acid functions with a suitable organic or inorganic acid or base, respectively. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2-hydroxyethanesulfonate, lactate, maleate, methanesulfonate, nicotinate, 2-naphth-alenesulfonate, oxalate, pamoate, pectinate, persulfate, 3-phenylproionate, picrate, pivalate, propionate, succinate, sulfate, tartrate, thiocyanate, p-toluenesulfonate, and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, hydroboric acid, nitric acid, sulfuric acid and phosphoric acid and such organic acids as formic acid, acetic acid, trifluoroacetic acid, fumaric acid, tartaric acid, oxalic acid, maleic acid, methanesulfonic acid, succinic acid, malic acid, methanesulfonic acid, benzenesulfonic acid, and p-toluenesulfonic acid, citric acid, and acidic amino acids such as aspartic acid and glutamic acid.

Basic addition salts can be prepared *in situ* during the final isolation and purification of the pyrimidine compounds, or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine, pyridine, picoline, triethanolamine and the like, and basic amino acids such as arginine, lysine and ornithine. The term "single formulation" as used herein refers to a single carrier or vehicle formulated to deliver effective amounts of both therapeutic agents to a patient. The single vehicle is designed to deliver an effective amount of each of the agents, along with any pharmaceutically acceptable carriers or excipients. In some embodiments, the vehicle is a tablet, capsule, pill, or a patch. In other embodiments, the vehicle is a solution or a suspension.

The term "unit dose" is used herein to mean simultaneous administration of both agents together, in one dosage form, to the patient being treated. In some embodiments, the unit dose is a single formulation. In certain embodiments, the unit dose includes one or more vehicles such that each vehicle includes an effective amount of at least one of the agents along with pharmaceutically acceptable carriers and excipients. In some embodiments, the unit dose is one or more tablets, capsules, pills, or patches administered to the patient at the same time.

The term "dose range" as used herein refers to an upper and a lower limit of an acceptable variation of the amount of agent specified. Typically, a dose of the agent in any amount within the specified range can be administered to patients undergoing treatment.

The term "treat" is used herein to mean to relieve, reduce or alleviate, at least one symptom of a disease in a subject. Within the meaning of the present invention, the term "treat" also denotes, to arrest, delay the onset (*i.e.,* the period prior to clinical manifestation of a disease or symptom of a disease) and/or reduce the risk of developing or worsening a symptom of a disease.

The term "subject" is intended to include animals. Examples of subjects include mammals, *e.g*., humans, dogs, cows, horses, pigs, sheep, goats, cats, mice, rabbits, rats, and transgenic non-human animals. In certain embodiments, the subject is a human, *e.g.,* a human suffering from, at risk of suffering from, or potentially capable of suffering from multiple myeloma.

The term "about" or "approximately" usually means within 20%, more preferably within 10%, and most preferably still within 5% of a given value or range. Alternatively, especially in biological systems, the term "about" means within about a log (*i.e.,* an order of magnitude) preferably within a factor of two of a given value.

The term "synergistic effect" as used herein, refers to action of two agents, i.e compound A, and dexamethasone, producing an effect, for example, slowing the symptomatic progression of multiple myeloma or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

An "effective amount" of a combination of agents ( compound A, and dexamethasone) is an amount sufficient to provide an observable improvement over the baseline clinically observable signs and symptoms of the depressive disorder treated with the combination.

An "oral dosage form" includes a unit dosage form prescribed or intended for oral administration.

### Medical Uses

The invention provides a combination for use in the treatment of multiple myeloma said combination comprising compound A, and dexamethasone. The term "myeloma" as used herein relates to a tumor composed of cells of the type normally found in the bone marrow. The term "multiple myeloma" as used herein means a disseminated malignant neoplasm of plasma cells which is characterized by multiple bone marrow tumor foci and secretion of an M component (a monoclonal immunoglobulin fragment), associated with widespread osteolytic lesions resulting in bone pain, pathologic fractures, hypercalcaemia and normochromic normocytic anaemia. Multiple myeloma is incurable by the use of conventional and high dose chemotherapies.

In some embodiments, the subject to be treated (*e.g.,* a human) is determined to be non responsive or resistant to one or.more multiple myeloma therapies.

Provided herein is a combination for use in the treatment of multiple myeloma said combination comprising compound A, and dexamethasone. The combination of agents is effective to treat the multiple myeloma. It is important to note the synergistic effects of the combination of agents: even though one or more of the agents administered alone at a particular dosage may not be effective when administered in combination, at the same dosage of each agent, the treatment is effective. The doses of the one or more of the agents in the combination therefore can be less than the FDA approved doses of each agent

### Dosage

The optimal dose of the combination of agents for use in the treatment of multiple myeloma can be determined empirically for each individual using known methods and will depend upon a variety of factors, including, though not limited to the degree of advancement of the disease; the age, body weight, general health, gender and diet of the individual; the time and route of administration; and other medications the individual is taking. Optimal dosages maybe established using routine testing and procedures that are well known in the art. For example, daily dosages for dexamethasone can be 0.25 mg - 9 mg (*e.g.,* 0.25, 0.5, 0.6, 0.75, 2 or 4 mg). Daily dosages for compound A can be 10 mg to about 2000 mg.

The amount of combination of agents that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone.

Frequency of dosage may vary depending on the compound used and the particular condition to be treated or prevented. In general, the use of the minimum dosage that is sufficient to provide effective therapy is preferred. Patients may generally be monitored for therapeutic effectiveness using assays suitable for the condition being treated or prevented, which will be familiar to those of ordinary skill in the art.

The dosage form can be prepared by various conventional mixing, comminution and fabrication techniques readily apparent to those skilled in the chemistry of drug formulations.

The oral dosage form containing the combination of agents or individual agents of the combination of agents may be in the form of micro-tablets enclosed inside a capsule, *e.g.* a gelatin capsule. For this, a gelatin capsule as is employed in pharmaceutical formulations can be used, such as the hard gelatin capsule known as CAPSUGEL, available from Pfizer.

Many of the oral dosage forms useful herein contain the combination of agents or individual agents of the combination of agents in the form of particles. Such particles may be compressed into a tablet, present in a core element of a coated dosage form, such as a taste-masked dosage form, a press coated dosage form, or an enteric coated dosage form, or may be contained in a capsule, osmotic pump dosage form, or other dosage form.

The drug compounds of the present invention ( compound A, and dexamethasone) are present in the combinations, dosage forms, pharmaceutical compositions and pharmaceutical formulations disclosed herein in a ratio in the range of 100:1 to 1:100, more preferably 1:1 to 1:100, and still more preferably 1:10 to 1:100.

The optimum ratios, individual and combined dosages, and concentrations of the drug compounds that yield efficacy without toxicity are based on the kinetics of the active ingredients' availability to target sites, and are determined using methods known to those of skill in the art.

### Pharmaceutical Compositions

The pharmaceutical compositions or combinations provided herein ( compound A, and dexamethasone) can be tested in clinical studies. Suitable clinical studies may be, for example, open label, dose escalation studies in patients with proliferative diseases. Such studies prove in particular the synergism of the active ingredients of the combination of the invention. The beneficial effects on proliferative diseases may be determined directly through the results of these studies which are known as such to a person skilled in the art. Such studies may be, in particular, be suitable to compare the effects of a monotherapy using the active ingredients and a combination of the invention. In one embodiment, the dose of compound A, is escalated until the Maximum Tolerated Dosage is reached, and dexamethasone is administered with a fixed dose. Alternatively, compound A may be administered in a fixed dose and the dose of dexamethasone may be escalated. Each patient may receive doses of compound A, either daily or intermittently. The efficacy of the treatment may be determined in such studies, *e*.*g*., after 12, 18 or 24 weeks by evaluation of symptom scores every 6 weeks.

The administration of a pharmaceutical combination of the invention may result not only in a beneficial effect, *e.g.* a synergistic therapeutic effect, *e.g.* with regard to alleviating, delaying progression of or inhibiting the symptoms, but also in further surprising beneficial effects, *e*.*g*. fewer side-effects, an improved quality of life or a decreased morbidity, compared with a monotherapy applying only one of the pharmaceutically active ingredients used in the combination of the invention.

A further benefit may be that lower doses of the active ingredients of the combination of the invention may be used, for example, that the dosages need not only often be smaller but may also be applied less frequently, which may diminish the incidence or severity of side- effects. This is in accordance with the desires and requirements of the patients to be treated.

It is one objective of this invention to provide a pharmaceutical composition comprising a quantity, which may be jointly therapeutically effective at targeting or preventing multiple myeloma. In this composition, compound A and dexamethasone may be administered together, one after the other or separately in one combined unit dosage form or in two separate unit dosage forms. The unit dosage form may also be a fixed combination.

The pharmaceutical compositions for separate administration of both compounds, or for the administration in a fixed combination, *i.e.* a single galenical composition comprising both compounds according to the invention may be prepared in a manner known *per se* and are those suitable for enteral, such as oral or rectal, and parenteral administration to mammals (warm-blooded animals), including humans, comprising a therapeutically effective amount of at least one pharmacologically active combination partner alone, *e.g.* as indicated above, or in combination with one or more pharmaceutically acceptable carriers or diluents, especially suitable for enteral or parenteral application.

### Formulations

In one embodiment, provided herein is a pharmaceutical composition comprising: (1) compound A, and/or a stereoisomer, tautomer, or pharmaceutically acceptable salt thereof, and (2) dexamethasone and/or a pharmaceutically acceptable salt, solvate, or racemate thereof, such that the subject is treated, wherein each of components (1) and (2) is formulated in admixture with a pharmaceutically-acceptable adjuvant, diluent or carrier.

The drug combinations provided herein may be formulated by a variety of methods apparent to those of skill in the art of pharmaceutical formulation. The various release properties described above may be achieved in a variety of different ways. Suitable formulations include, for example, tablets, capsules, press coat formulations, and other easily administered formulations.

Suitable pharmaceutical formulations may contain, for example, from about 0.1 % to about 99.9%, preferably from about 1 % to about 60 %, of the active ingredient(s). Pharmaceutical formulations for the combination therapy for enteral or parenteral administration are, for example, those in unit dosage forms, such as sugar-coated tablets, tablets, capsules or suppositories, or ampoules. If not indicated otherwise, these are prepared in a manner known *per se,* for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount may be reached by administration of a plurality of dosage units.

In particular, a therapeutically effective amount of each of the combination partner of the combination of the invention may be administered simultaneously or sequentially and in any order, and the components may be administered separately or as, a fixed combination. For example, the combination for use in the treatment of multiple-myeloma according to the invention may comprises (i) the first agent (a) in free or pharmaceutically acceptable salt form being administered and (ii) an agent (b) in free or pharmaceutically acceptable salt form, being administered simultaneously or sequentially in any order, in jointly therapeutically effective amounts, preferably in synergistically effective amounts, *e.g.* in daily or intermittently dosages corresponding to the amounts described herein. The individual combination partners of the combination of the invention may be administered separately at different times during the course of therapy or concurrently in divided or single combination forms. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single active ingredients required to alleviate, counter or arrest the progress of the condition.

### Examples

The invention is further illustrated by the following examples. The examples should not be construed as further limiting.

### Materials and methods

### Cell lines, primary myeloma cells, bone marrow stromal cells (BMSCs), peripheral blood mononuclear cells (PBMCs), antibodies and reagents

MM cell lines ARP1, ARK, MM.1S, MM.1R and U266 were maintained in RPMI-1640 medium supplemented with 10% fetal bovine serum, 100 U/ml penicillin and 100 µg/ml streptomycin at 37°C and 5% CO₂. Primary MM cells and MM BMSCs were isolated from bone marrow aspirates of myeloma patients. PBMCs were obtained from healthy volunteers. The study was approved by the Institutional Review Board at The University of Texas M.D. Anderson Cancer Center. Anti-caspase-3, caspase-9, PARP, Bim, XIAP, cyclin D1, pp70S6K (Thr389) and p27(Kip1) antibodies were purchased from Cell Signaling. Anti-bcl-2, Bcl-XL, Akt, pAkt (Thr 308), pAkt (Ser 473), p70S6K and β-actin antibodies were purchased from Santa Cruz. Compound A was dissolved in DMSO at 10 mM as stock solution. Dexamethasone and Propidium iodide (PI) were purchased from Sigma-Aldrich. Recombinant human IL6 was purchased from R&D Systems. FITC conjugated Annexin V was obtained from Invitrogen.

### Preparation of 5-(2,6-di-morpholin-4-yl-pyrimidin-4 yl)-4-trifluoromethyl-pyridin-2-ylamine (Compound A)

To a slurry of 2-morpholino-4,6-dichloropyrimidine (2.0 g, 8.54 mmol) in NMP (14 mL), triethylamine (1.43 mL, 10.25 mmol) was added. The heterogeneous mixture was stirred for 15 minutes, then treated with morpholine (0.75 mL, 8.54 mmol). Upon refluxing at 85 °C under argon for 2 hours, the solution was cooled, then added to EtOAc (160 mL). The organic solution was washed with 25 mL of NaHCO₃(sat.) (2 x), water (2 x) and brine, dried over Na₂SO₄, filtered and concentrated. The crude material was dissolved in 200 mL EtOAc and filtered through a SiO₂ pad, further eluting with EtOAc, yielding 2.2 g (93%) of 2,4-dimorpholino-6-chloropyrimidine as an off-white solid. LCMS (m/z): 285.0 (MH+), ¹H NMR (CDCl₃): δ 5.86 (s, IH), 3.71-3.76 (m, 12H), 3.52-3.56 (m, 4H).

Argon gas was bubbled through a heterogeneous mixture of 2,4-dimorpholino-6-chloropyrimidine (4.1 g, 14.3 mmol) and 4-(trifluoromethyl)-5-(4,4,5,5-tetramethyl-1,3,2-dioxaborolan-2-yl)pyridm-2-amine (16.5 g, 57.3 mmol) in 1,2-dimethoxyethane and 2M Na2Cθ3 (3:1) for 20 minutes. 1,1'-Bis(diphenylphosphino)ferrocene palladium (II) chloride (292 mg, 0.36 mmol) was added and the high pressure glass vessel containing the mixture was sealed. The reaction mixture was then heated at 90 °C for 15 hours, cooled and diluted with EtOAc (300 mL). The organic solution was washed with 300 mL of a mixture of water: Na₂CO₃(sat.):NH₄OH(conc.) = 5:4:1, then NH₄Cl(sat), and brine (2x), dried over Na₂SO₄, filtered and concentrated. The crude material was purified by SiO₂ chromatography (50- 90% EtOAc/hexanes with 0.1% TEA) resulting in 5.62 g (95%) of 5-(2,6-di-morpholin-4-yl-pyrimidin-4-yl)-4-trifluoromethyl-pyridin-2-ylamine as an off-white solid. LCMS (m/z): 411.3 (MH+); ¹H NMR (CDCl₃): δ 8.27 (s, IH), 6.78 (s, IH), 5.97 (s, IH), 4.77 (bs, 2H), 3.59-3.80 (m, 12H), 3.58-3.61 (m, 4H).

### Cell growth assay

The growth inhibitory effects of compound A on multiple myeloma (MM) cell lines were assessed by MTS assay following the manufacture's protocol (Promega). In brief, MM cells were plated in 96-well plates at a concentration of 5,000 cells/100 µl medium per well and treated with 0 to 1 mM final concentrations of compound A for 24 or 72 hours. By end of the assay, 20 µl of MTS/PMS solution was added into the culture medium in each well. The plates were then incubated for 4 hours at 37 °C and 5% CO₂. The absorbance at 490 nm was recorded using an ELISA plate reader. All experiments were performed in triplicate.

### Apoptosis assays

Compound A-induced cell apoptosis was detected by Annexin V binding assay as previously described (Zheng Y, Cai Z, Wang S, et al. Macrophages are an abundant component of myeloma microenvironment and protect myeloma cells from chemotherapy drug-induced apoptosis. Blood. 2009; 114 (17):3625-3628). Briefly, MM cells were cultured in 24-well plates and treated with 0 to 1 mM final concentrations of compound A for 24 or 72 hours. The cells were washed twice with cold PBS and resuspended in Annexin V binding buffer (Invitrogen). MM cells were stained with FITC-conjugated Annexin V and propidium iodide (PI) for 15 min at room temperature. Apoptotic cells were determined as Annexin V positive cells.

### Cell cycle analysis

MM cell lines ARP1, MM.1S and MM.1R were cultured with or without 1 µM compound A for 24 hours. Then, cells were harvested and permeabilized in 70% ethanol at 4 °C for overnight, and incubated with 50 µg/ml PI and 20µg/ml RNase A for 15 min. DNA content was analyzed by flow cytometry and FlowJo software.

### In vivo effects of compound A on established multiple myeloma

Six to eight week old female SCID mice were housed and monitored in MD. Anderson Cancer Center animal research facility. All experimental procedures and protocols had been approved by the Institutional Animal Care and Use Committee at The University of Texas M.D. Anderson Cancer Center. SCID mice were subcutaneously inoculated in the right flank with 1 million ARP1 cells suspended in 50 µl PBS. After palpable tumor developed (tumor diameters ≥ 5 mm), mice were treated with daily intraperitoneal injection of PBS or compound A (5 µmol per kg per day). Tumor sizes were measured every 5 days, and blood samples were collected with the same period. Tumor burdens were evaluated by measuring tumor size and detecting circulating human kappa chain.

### ELISA

Levels of human kappa chain in mice serum were measured by a quantitative ELISA (Bethyl Laboratories Inc) followed the vendor's protocol.

### Statistical analysis

All data are shown as mean ± standard deviation. The Student t test was used to compare various experimental groups. Significance was determined when P<0.05.

### Results

### Compound A inhibits growth of MM cell lines and induces the cell apoptosis

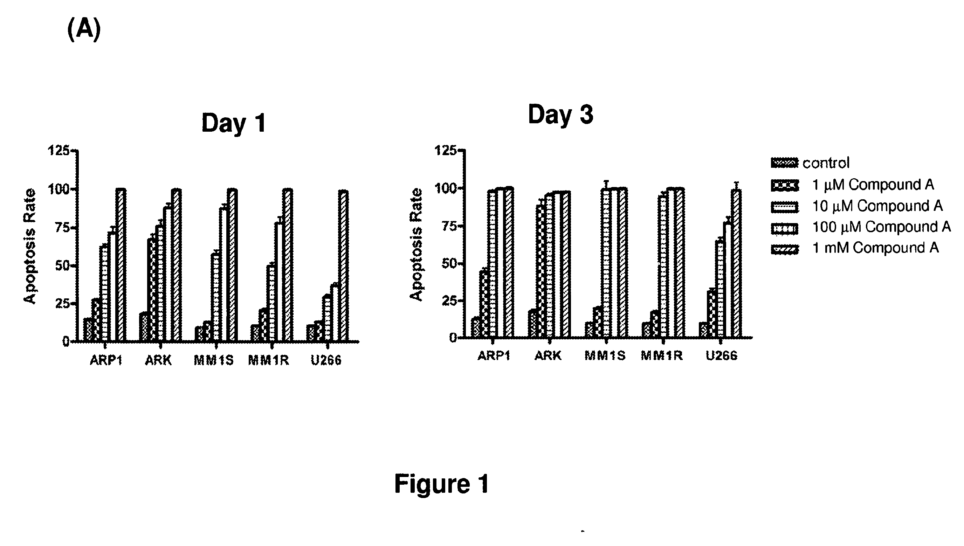

To evaluate the effect of compound A on myeloma cells, MM cell lines ARP1, ARK, MM.1S, MM.1R and U266 were treated with different doses of compound A for 24 hours or 72 hours. Compound A induced MM cells apoptosis were measured as described in *Materials and Methods.* As shown in Fig 1A, compound A induced MM cells apoptosis in both a dose dependent and a time dependent manner. Different MM cell lines had different sensitivities toward compound A. U266 was less sensitive to compound A compared to other MM cell lines. Compound A at concentration equal or greater than 10 µM induced significant apoptosis in all tested MM cell lines at 24 hours (P<0.05, compared with control). Therefore, 10 µM compound A 24 hours treatment was used in the following experiments.

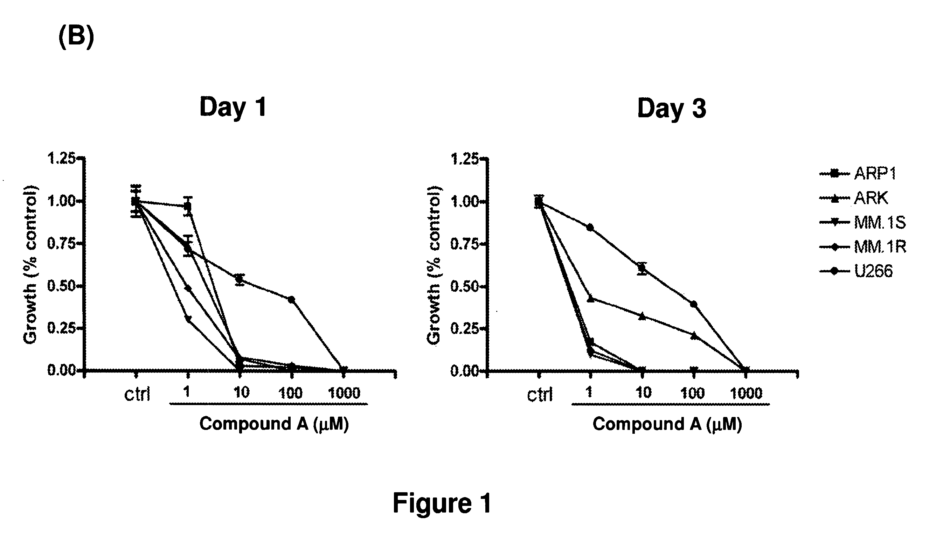

The effect of compound A on MM cells growth was tested by MTS assay. As shown in Fig 1B, compound A treatment resulted in dose dependent growth inhibition in all tested MM cell lines. Compound A IC₅₀ (concentration at 50% inhibition) varied in tested MM cells. At 24 hours treatment, IC₅₀ for ARP1, ARK and MM.1R was between 1 µM to 10 µM, while IC₅₀ for MM.1S was less than 1 µM and IC₅₀ for U266 was between 10 µM to 100 µM. In summary, these findings indicate that compound A treatment resulted in MM cells growth inhibition and apoptosis in a compound A dose dependent manner.

### Compound A induces primary MM cells apoptosis ex vivo

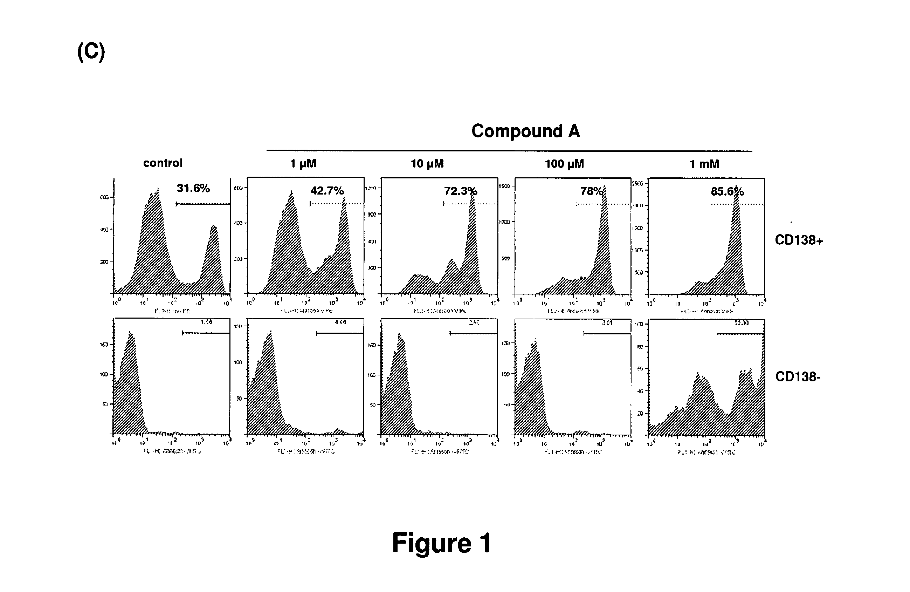

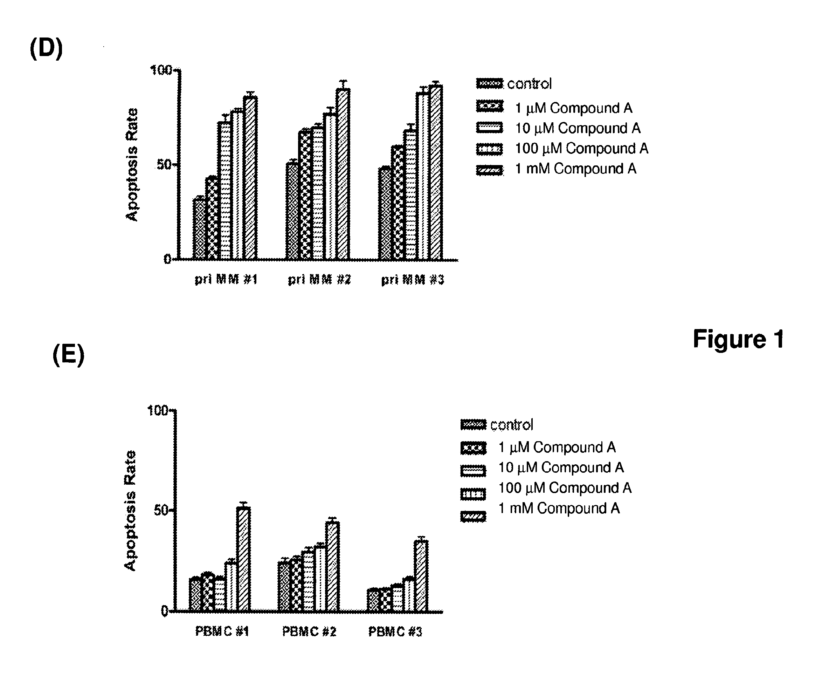

To evaluate compound A function in primary MM cells, the study was extended to CD138+ primary MM cells freshly isolated from myeloma patients. According to the previous finding, primary MM cells undergo apoptosis *ex vivo* unless the cells are cocultured with BMSCs (Zheng Y, Cai Z, Wang S, et al. Macrophages are an abundant component of myeloma microenvironment and protect myeloma cells from chemotherapy drug-induced apoptosis. Blood. 2009;114 (17):3625-3628). Therefore, CD 138+ primary MM cells were cocultured at 1:1 ratio with CD 138- BMSCs isolated from MM bone marrow aspiration. The cells were treated with different doses of compound A from 0 to 1 mM for 24 hours. The primary MM cells and BMSCs were separated by APC-CD138 staining. As shown by the representative data obtained from myeloma cells and BMSCs from one out of three patients examined (Fig 1C), compound A induced primary MM cells (CD138+) apoptosis in a dose dependent manner. 10 µM compound A induced more than 70% of primary MM cells apoptosis. Of interest, compound A had significantly lower cytotoxicity toward CD 138- stromal cells. Fig 1D shows compound A induced apoptosis of primary MM cells from three different MM patients. Given together, these data indicate that compound A induces primary MM cells apoptosis, but has low toxicity toward non-tumeric BMSCs.

### Compound A has low toxicity toward normal blood cells of healthy volunteers

To examine whether compound A induces normal PBMCs apoptosis, normal PBMCs from different healthy volunteers were incubated with 0 to 1 mM compound A for 24 hours. Cells apoptosis rate were measured as described above. As shown in Fig 1E, compound A had comparably low toxicity toward normal PBMCs. Effective compound A concentration to MM cells, 10 µM or 100 µM, only resulted in less than 40% of PBMC apoptosis. Thus, these findings indicate that compound A has low cytotoxicity toward resting PBMCs.

### Figure 1 Legend

(A). Five MM cell lines ARP1, ARK, MM.1S, MM.1R and U266 were cultured in the presence of 0 to 1 mM compound A. Cells were harvested after 1 day or 3 days of treatment. Compound A induced MM cell apoptosis were measured by Annexin V staining as described in *Materials and Methods.* (B). Same MM cell lines were treated with 0 to 1 mM compound A for 1 or 3 days. Cell growths were assessed by MTS assay. (C).Representative histograms of primary MM cells treated with compound A. (D). Data from 3 patients showing a dose-dependent induction of apoptosis in freshly isolated primary MM cells. Cells were treated with different doses of compound A for 1 day. (E). PBMCs isolated from 3 healthy volunteers were treated with compound A for 1 day.

### IL-6 addition or presence of BMSCs does NOT protect MM cells from compound A induced apoptosis

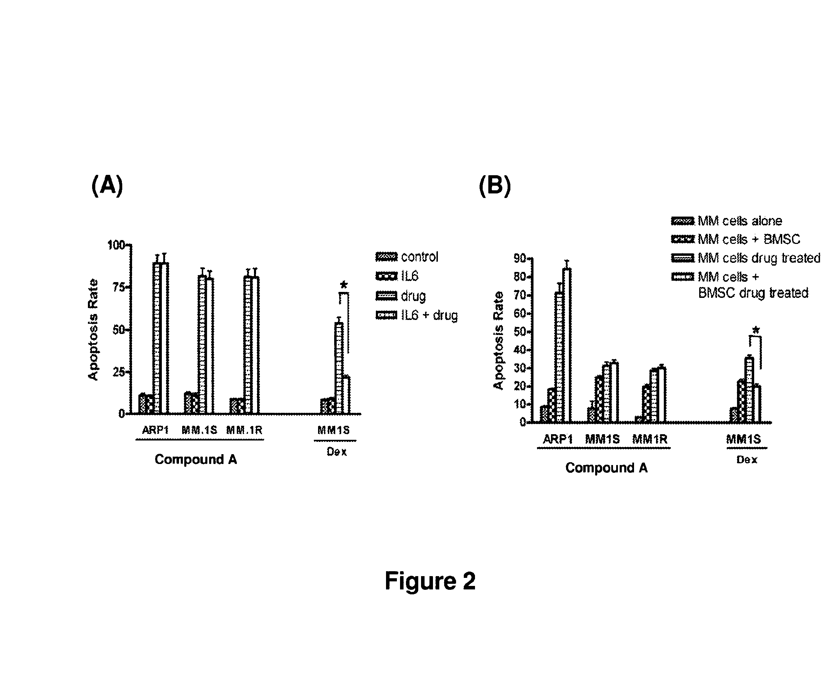

IL-6 is an important survival cytokine for MM (Klein B, Zhang XG, Jourdan M, et al. Interleukin-6 is the central tumor growth factor in vitro and in vivo in multiple myeloma. Eur Cytokine Netw. 1990;1 (4):193-201; Gado K, Domjan G, Hegyesi H, Falus A. Role of INTERLEUKIN-6 in the pathogenesis of multiple myeloma. Cell Biol Int. 2000;24 (4):195-209). Previous work has shown that IL-6 promotes MM cell survival under chemotherapy agent dexamethasone treatment (Frassanito MA, Cusmai A, Iodice G, Dammacco F. Autocrine interleukin-6 production and highly malignant multiple myeloma: relation with resistance to drug-induced apoptosis. Blood. 2001;97 (2):483-489). Therefore, attenuation of compound A-induced MM cell apoptosis by IL6 addition was examined. For this purpose, different MM cell lines ARP1, MM.1S and MM.1R were cultured with or without recombinant hIL-6 (final concentration 5 µg/ml) and treated with or without 10 µM compound A for 2 days. As a positive control, MM.1S cells were treated with 40µg/ml of dexamethasone with or without hIL-6 addition for the same period of time. As shown in Fig 2A, IL6 addition did not affect compound A induced MM cell apoptosis, but promoted MM.1S cells survival under dexamethasone treatment.

Mounting evidence has shown that bone marrow stromal cells (BMSCs) in myeloma tumor bed provide a tumor promotion microenvironment and protect MM cells from chemotherapy drugs induced apoptosis (De Raeve HR, Vanderkerken K. The role of the bone marrow microenvironment in multiple myeloma. Histol Histopathol. 2005;20 (4):1227-1250; Mitsiades CS, McMillin DW, Klippel S, et al. The role of the bone marrow microenvironment in the pathophysiology of myeloma and its significance in the development of more effective therapies. Hematol Oncol Clin North Am. 2007;21 (6):1007-1034, vii-viii). Therefore, it was also tested whether BMSCs from MM patient bone marrow are able to protect MM cells from compound A-induced cell apoptosis. For this purpose, BMSCs isolated from MM patient were cocultured with MM cell lines ARP1, MM.1S and MM.1R. The cells were treated with or without 10 µM of compound A for 24 hours. As a positive control, MM.1S cells were cocultured with or without BMSC and treated with or without 40µg/ml of dexamethasone for 24 hours. After treatment, the MM cells were identified as CD138+ cells by APC-CD138 staining. As shown in Fig 2B, BMSCs did not protect compound A induced MM cell apoptosis, but protected MM.1S cells from dexamethasone induced cell apoptosis.

### Figure 2 Legend

(A). MM cell lines ARP1, MM.1S and MM.1R were cultured with 10 µM compound A for 3 days. rhIL-6 were added to final concentration of 5 ng/ml. As a positive control, MM.1S cells were treated with 40 µg/ml dexamethasone in the presence of 5 ng/ml of rhIL6 for the same time. Cells apoptosis rate was measured by annexin V staining. (B). Same MM cells were co-cultured with or without MM BMSC, and treated with 10 µM of compound A for 1 day. As a positive control, MM.1S cells were cocultured with or without BMSC and treated with 40 µg/ml dexamethasone for 1 day. CD138+ MM cells apoptosis rate was measured by Annexin V staining.

### Compound A causes cell cycle arrest in G1 phase

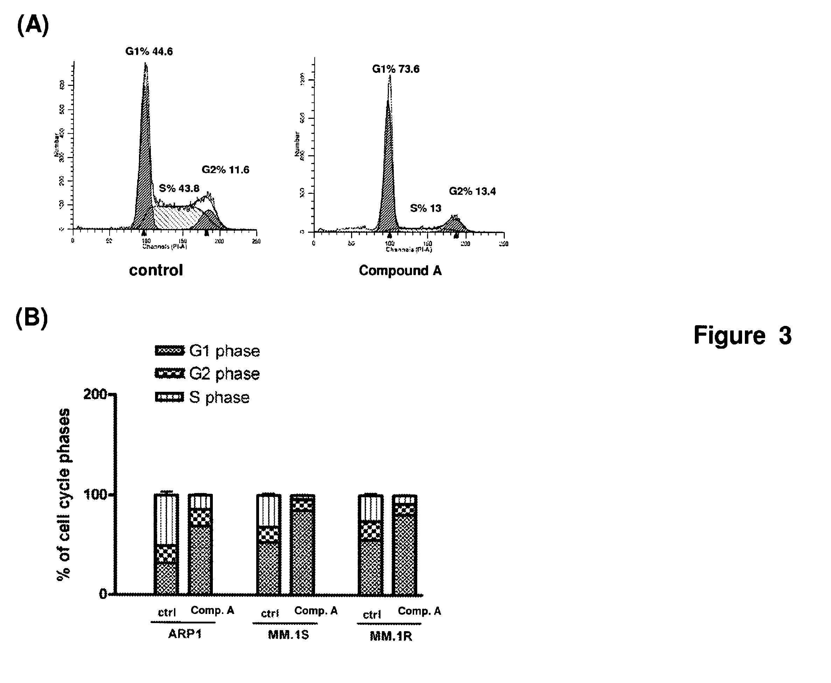

To study the mechanism of compound A induced MM cells growth inhibition and apoptosis, it was examined whether compound A treatment affects MM cell cycle. As shown in Fig 3A, ARP1 cells were cultured with or without 1µM compound A for 24 hours. Compound A treatment resulted in increased G1 phase cells and decreased S phase cells. Similar findings were observed in other MM cell lines MM.1S and MM.1R (Fig. 3B).

### Figure 3 Legend

(A). Representative histogram of ARP1 treated with 1 µM compound A for 1 day. (B). MM cells ARP1, MM.1S and MM.1R were treated with 1 µM of compound A for 1 day. Cell cycle was tested as described.

### Compound A triggers MM cells apoptosis by caspases activation

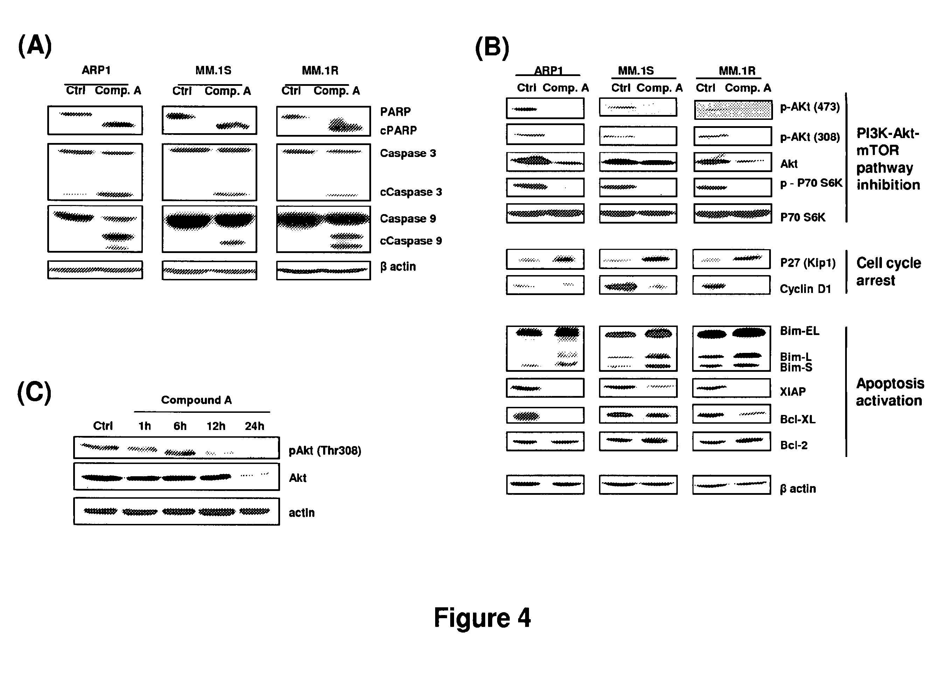

To elucidate compound A induced MM cell apoptosis, MM cell lines ARP1, MM.1S and MM.1R, treated with or without compound A for 24 hours, were assessed by Western blotting analysis. The result showed the cleavage of caspase 3 and caspase 9 (Fig. 4A).

PARA cleavage was also detected after compound A treatment in all tested cell lines. Overall, these findings indicate that compound A treatment induces MM cells apoptosis through caspases activation.

### Compound A exposure causes up-regulation of BimS and down-regulation of XIAP

To further analyze the signaling pathways that were affected by compound A exposure in MM cells, immunoblotting was extended to other cell signaling molecules. First, the inhibitory effect of compound A on PI3K-Akt-mTOR pathway in MM cells was tested. As shown in Fig 4B, both p-Akt at Thr473 and Ser308 were down-regulated after compound A treatment. The total Akt levels were also decreased in ARP1 and MM.1R cells after compound A treatment (Fig 4C). This is probably because of increased apoptotic cells after compound A treatment. The p-P70S6K levels were also decreased after compound A treatment in tested MM cell lines, while total P70S6K expression remained constant. Such findings indicate that compound A inhibits PI3K-Akt-mTOR pathways in MM cells.

Second, since compound A treatment caused cell cycle arrest in G1 phase, the expression of cell cycle regulators was tested. As shown in Fig 4B, cell cycle repressor p27 (Kip1) protein expression was up-regulated after compound A treatment, while cyclin D1 expression was down-regulated.

Next, the expressions of apoptosis regulatory factors was tested. Our data showed that the cytotoxic small isoforms of Bim, BimS expression was up-regulated after compound A treatment. Bim is a pro-apoptotic factor belonging to Bcl-2 family (O'Connor L, Strasser A, O'Reilly LA, et al. Bim: a novel member of the Bcl-2 family that promotes apoptosis. EMBO J. 1998;17 (2):384-395). Bim has three major isoforms generated by alternative splicing, BimEL, BimL and BimS. The shortest form BimS is the most cytotoxic isoform (Weber A, Paschen SA, Heger K, et al. BimS-induced apoptosis requires mitochondrial localization but not interaction with anti-apoptotic Bcl-2 proteins. J Cell Biol. 2007; 177 (4):625-636). Previous work has shown that the transcription of Bim is regulated by the forkhead transcription factor FKHR-L1, a downstream effector of PI3K (Dijkers PF, Medema RH, Lammers JW, Koenderman L, Coffer PJ. Expression of the pro-apoptotic Bcl-2 family member Bim is regulated by the forkhead transcription factor FKHR-L1. Curr Biol. 2000;10 (19):1201-1204). In addition to Bim, XIAP and Bcl-XL, both are anti-apoptotic proteins (Deveraux QL, Roy N, Stennicke HR, et al. IAPs block apoptotic events induced by caspase-8 and cytochrome c by direct inhibition of distinct caspases. EMBO J. 1998; 17 (8):2215-2223; Minn AJ, Kettlun CS, Liang H, et al. Bcl-xL regulates apoptosis by heterodimerization-dependent and -independent mechanisms. EMBO J. 1999;18 (3):632-643) expressions were down-regulated after compound A treatment. Thus, compound A induced MM cell apoptosis may be caused by up-regulation of cytotoxic BimS and down-regulation of anti-apoptotic XIAP and Bcl-XL.

### Figure 4 Legend

(A). MM cells ARP1, MM.1S and MM.1R were treated with or without 10 µM compound A for 1 day. Compound A induced activation and cleavage of caspase-3, caspase-9 and PARP is shown. (B). Same MM cells treated with 10 µM compound A were lysed for western blotting. (C) RP1 cells were treated with 10 µM compound A for 1,6, 12 and 24 hours.

### Synergistic cytotoxicity of compound A and dexamethasone combined treatment on MM cells

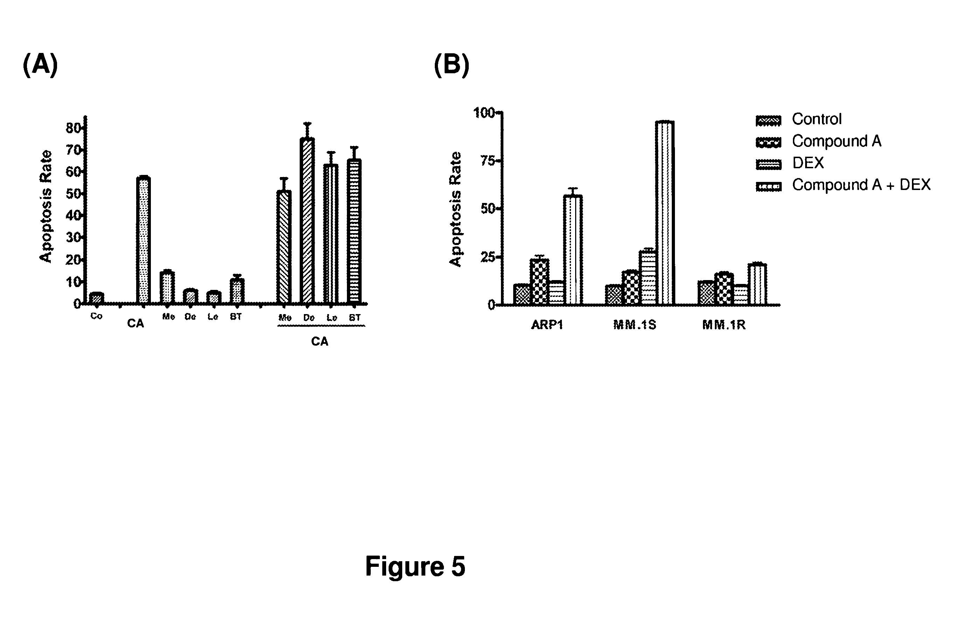

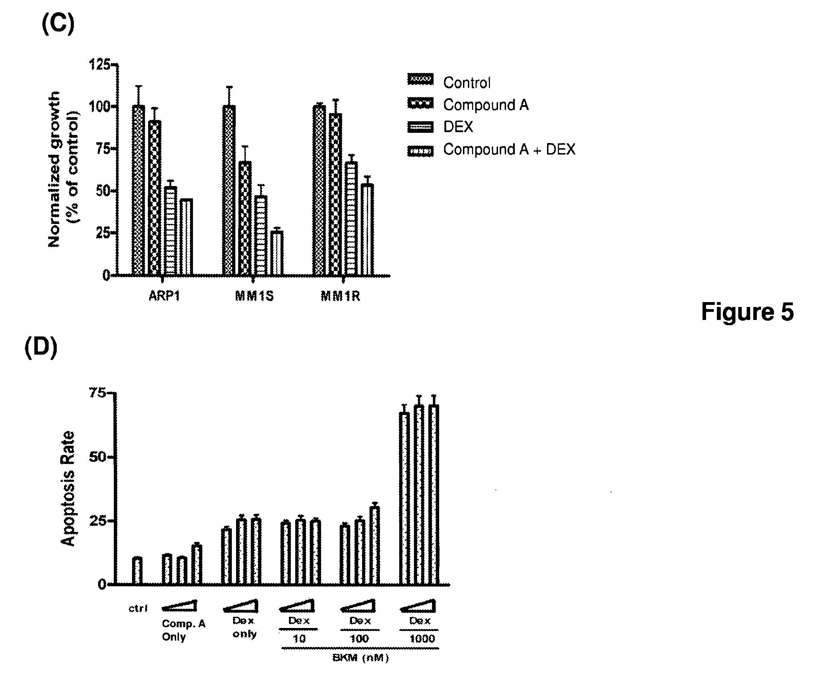

To test whether compound A has a synergistic/additive effect with other MM chemotherapy agents, ARP1 cells were treated with compound A in combination with melphalan, dexamethasone, lenalidomide and bortezomib. As shown in Fig 5A, compound A and dexamethasone combined treatment had synergistic/additive cytotoxicity in ARP1 cells. Next, the experiment was extended to other MM cell lines, and they were treated with low compound A (1 µM) and dexamethasone (40 µg/ml). As shown in Fig 5B, although low doses of compound A or dexamethasone alone had only limited cytotoxic effect, combined dual-drug treatment induced significant cell apoptosis in dexamethasone sensitive cell lines ARP1 and MM.1S, but not in dexamethasone resistant cells MM.1R. Cell growth test also showed that compound A and dexamethasone synergistically inhibited MM.1S cell growth (Fig 5C).

To examine the minimum doses of each drug for synergistic effect, MM.1S cells were treated with different doses of compound A and dexamethasone for 24 hours. As shown in Fig 5D, 1 µM compound A was necessary for synergistic effect, while as low as 40 ng/ml dexamethasone was enough to synergistically stimulate cell apoptosis. Increasing dexamethasone dosage did not increase cell death rate.

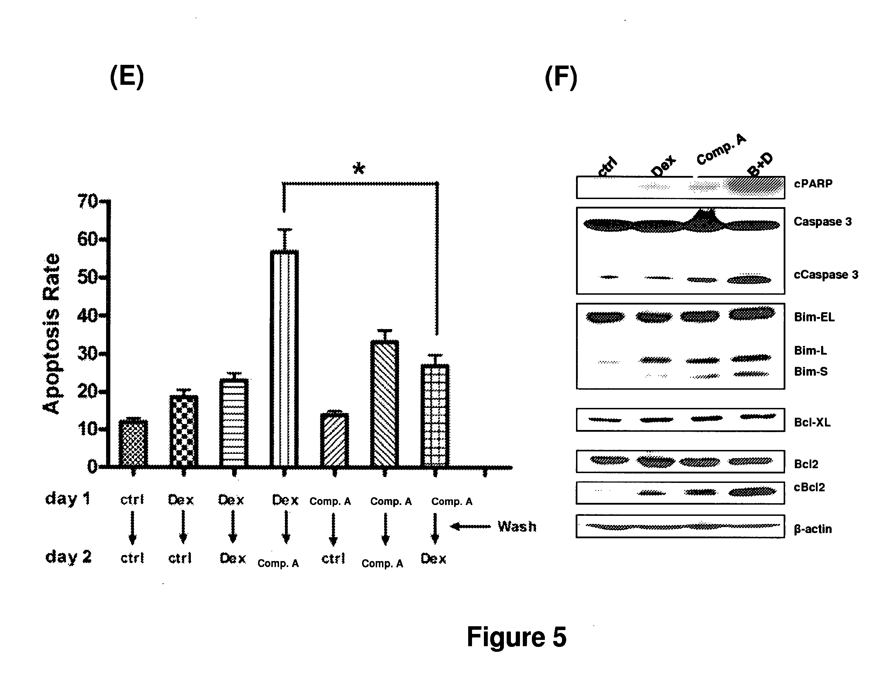

To elucidate the role of compound A and dexamethasone in the synergistic effect on MM.1S cells, the cells were treated with drugs in order. In specific, MM.1S cells were treated with dexamethasone for the first day, and switched to compound A treatment for the second day, or the reverse. Cells apoptosis rates were measured after the second day. As shown in Fig 5E, dexamethasone followed by compound A treatment resulted in a higher apoptosis rate than any other kinds of treatment.

At last, the synergistic effect was tested by immunoblotting. As shown in Fig. 5F, compound A and dexamethasone co-treatment resulted in increased PARP cleavage, Bcl-2 cleavage and caspase 3 activation. These findings indicate an enhanced apoptosis after dual drugs treatment. BimS expression was up-regulated in combined treatment, which may be the cause of synergistic effect. In summary, these findings indicate that the combination of compound A and dexamethasone has synergistic cytotoxicity in dexamethasone-sensitive MM cells.

### Figure 5 Legend

(A). ARP1 cells were treated with 10 µM compound A (BK), 15 nM melphalan (Me), 40 µg/ml dexamethasone (De), 100 µM lenalidomide (Le), 10 µM Bortizomib (BT), or their combinations for 1 day. The cell apoptosis were measured as described. (B). MM cells ARP1, MM.1S and MM.1R were treated with 1 µM compound A, 40 µg/ml dexamethasone or their combination for 1 day. Cell apoptosis was measured. (C). Same MM cell lines were treated with compound A for 1 day and cell growth were measured by MTS. (D). MM.1S cells were treated with different dose of compound A (10 nM, 100 nM and 1000 nM), dexamethasone (40 ng/ml, 400 ng/ml and 4000 ng/ml) or their combination for 1 day. Cell apoptosis were measured by annexin V staining. (E). MM.1S cells were treated with 1 µM compound A or 4 µg/ml dexamethasone for 1 day. Then cells were washed once with PBS and switch to second condition medium containing either 1 µM compound A or 4 µg/ml dexamethasone for another 24 hours. Cells apoptosis rates were measured by annxin V staining. (F). MM.1S cells were treated with dexamethasone (4 ug/ml), compound A (1 µM) or both for 24 hours.

### In vivo effects of compound A on established MM

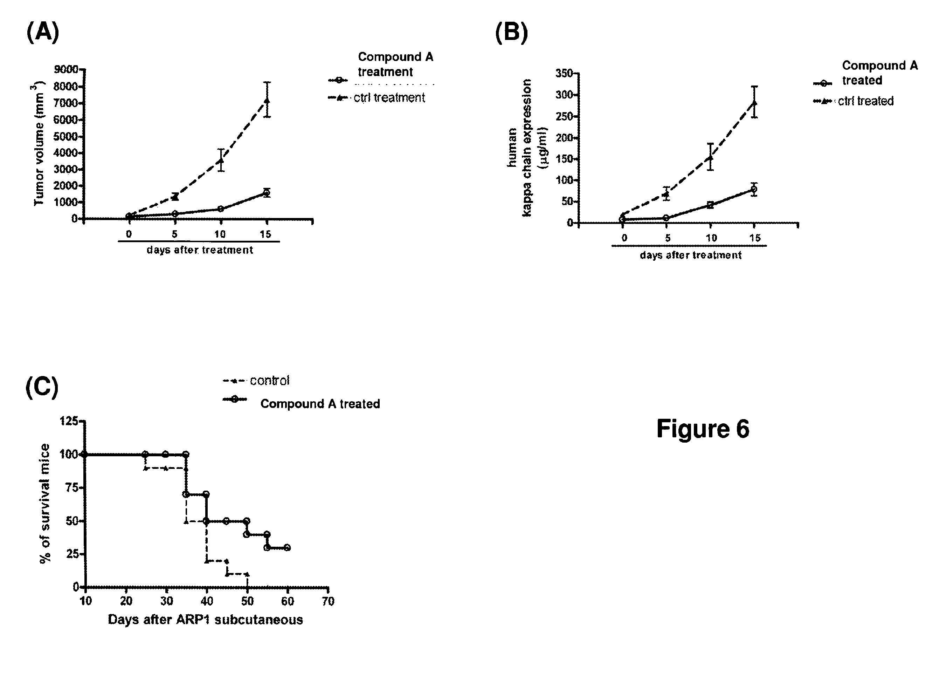

To examine compound A anti-MM effects *in vivo,* human MM model in SCID mice were established as described in *Materials and Methods.* When palpable tumors developed (≥5 mm diameters), mice (10 per group) received intraperitoneal injection of compound A (5 µmol per kg per day) or vehicle control PBS daily. As shown in Fig. 6A and 6B, mice that received compound A treatment had significantly smaller tumor burdens compared to control mice, which was measured by tumor volume (Fig. 6A, P<0.05) and level of circulating human kappa chain (Fig 6B, P<0.05). In addition, compound A treatment significantly prolonged the survival of tumor-bearing mice (Fig 6C). Thus, these data demonstrate the anti-MM capacity of compound *A in vivo.*

### Figure 6 Legend

SCID mice were inoculated subcutaneously in the right flank with 1 X 10⁶ ARP1 cells. Three to 4 weeks later when palpable tumors (≥ 5 mm in diameter) developed, mice (10 per group) were treated with intraperitoneal injections of PBS or compound A (100 nmol per mouse per day) daily. Tumor burdens were measured as (A) tumor volumes and (B) levels of circulating human kappa china in SCID mice sera detected by ELSA, and (C) survival of tumor-bearing mice.

### Discussion

Multiple myeloma (MM) is still an incurable disease with median survival only about 5 years (Kumar SK, Rajkumar SV, Dispenzieri A, et al. Improved survival in multiple myeloma and the impact of novel therapies. Blood. 2008;111 (5):2516-2520). Therefore, new therapeutic agents are needed in MM treatment. The excessive activation of PI3K-Akt pathway in MM has been reported (Pene F, Claessens YE, Muller O, et al. Role of the phosphatidylinositol 3-kinase/Akt and mTOR/P70S6-kinase pathways in the proliferation and apoptosis in multiple myeloma. Oncogene. 2002;21 (43):6587-6597). IGF-1 and IL6, the two major growth factors in MM, promote myeloma cell proliferation and drug resistance by activating PI3K-Akt pathway (Hideshima T, Nakamura N, Chauhan D, Anderson KC. Biologic sequelae of interleukin-6 induced PI3-K/Akt signaling in multiple myeloma. Oncogene. 2001;20 (42):5991-6000; Mitsiades CS, Mitsiades NS, McMullan CJ, et al. Inhibition of the insulin-like growth factor receptor-1 tyrosine kinase activity as a therapeutic strategy for multiple myeloma, other hematologic malignancies, and solid tumors. Cancer Cell. 2004;5 (3):221-230). A panel of PI3K-Akt-mTOR pathway inhibitors has been shown to exhibit anti-MM activities *both in vitro* and *in vivo* (McMillin DW, Ooi M, Delmore J, et al. Antimyeloma activity of the orally bioavailable dual phosphatidylinositol 3-kinase/mammalian target of rapamycin inhibitor NVP-BEZ235. Cancer Res. 2009;69 (14):5835-5842; Ikeda H, Hideshima T, Fulciniti M, et al. PI3K/p110{delta} is a novel therapeutic target in multiple myeloma. Blood*;* Cirstea D, Hideshima T, Rodig S, et al. Dual inhibition of akt/mammalian target of rapamycin pathway by nanoparticle albumin-bound-rapamycin and perifosine induces antitumor activity in multiple myeloma. Mol Cancer Ther;9 (4):963-975). Therefore, PI3K-Akt-mTOR pathway targeting therapy is a promising way to treat MM.

In this study, the anti-MM activity of compound A, a pan-PI3K inhibitor, has been demonstrated. Compound A treatment results in cell growth inhibition and apoptosis induction in all tested MM cell lines and primary MM cells in a dose dependent manner (Fig.1A, 1B, 1C, 1D and 2A). Compound A has only limited cytotoxicity toward normal PBMCs or non-malignant BMSCs (Fig 1C, 1E). In addition, compound A shows anti-MM activity *in vivo* in MM model in SCID mice. Compound A treated MM bearing mice has repressed tumor growth and prolonged survival (Fig. 6A, 6B and 6C). Of importance, compound A induced MM cytotoxicity overcomes the drug resistance provided by the presence of BMSCs or IL-6 (Fig 2A, 2B). Previous research has shown that BMSCs in MM bone marrow play a crutial role in MM drug resistance (Epstein J, Yaccoby S. Consequences of interactions between the bone marrow stroma and myeloma. Hematol J. 2003;4 (5):310-314; Dalton WS. Drug resistance and drug development in multiple myeloma. Semin Oncol. 2002;29 (6 Suppl 17):21-25). One mechanism of BMSCs mediated drug resistance is that BMSCs secret drug resistant factor IL-6 and protect MM cells from chemotherapy induced apoptosis (Hideshima T, Nakamura N, Chauhan D, Anderson KC. Biologic sequelae of interleukin-6 induced PI3-K/Akt signaling in multiple myeloma. Oncogene. 2001;20 (42):5991-6000). As a result, MM patients usually generate drug tolerance to conventional chemotherapy agents during the treatment and have relapse tumor that is more resistant to the drugs (Kastritis E, Palumbo A, Dimopoulos MA. Treatment of relapsed/refractory multiple myeloma. Semin Hematol. 2009;46 (2): 143-157). Thus, these findings indicate that compound A has potent anti-MM activity and is of benefit to those MM patients who are resistant to conventional chemotherapy drugs.

The signaling transduction and downstream effectors of PI3K-Akt-mTOR pathway have been studied in different cancer cell models. In general, PI3K-Akt inhibition results in cell cycle arrest, cell growth repression and apoptosis (Sasaki T, Takasuga S, Sasaki J, et al. Mammalian phosphoinositide kinases and phosphatases. Prog Lipid Res. 2009;48 (6):307-343; Di Paolo G, De Camilli P. Phosphoinositides in cell regulation and membrane dynamics. Nature. 2006;443 (7112):651-657).

In the disclosed experiments, the inhibitory effect of compound A on PI3K-Akt-mTOR pathway in MM cell lines have been demonstrated (Fig 3B and 3C). Compound A causes MM cells cell cycle arrest at G1 phase by up-regulation of p27 (Kip1) and down-regulation of cyclin D1 (Fig 3A, 3B and 4B). In addition, compound A exposure results in up-regulation of apoptotic BimS expression and down-regulation of anti-apoptotic XIAP expression, both of which may cause MM cell apoptosis.

These findings indicate a synergistic anti-MM activity of the combination of compound A and dexamethasone (Fig 5A, 5B). This synergistic effect only exists in dexamethasone-sensitive cells, but not in dexamethasone-resistant cells. More important, the combination of compound A and dexamethasone exhibits a synergistic effect even with low doses of each drug (Fig 5B, 5D). In addition, the combination of compound A treatment after dexamethasone exposure shows enhanced anti-MM activity as well, compared to treatment with dexamethasone or compound A only (Fig 5E). Dexamethasone is widely used in MM treatment, alone or together with other chemotherapeutic drugs (Ludwig H, Beksac M, Blade J, et al. Current multiple myeloma treatment strategies with novel agents: a European perspective. Oncologist; 15 (1):6-25). The general rule for dexamethasone usage is to give the smallest dose necessary to result a good response and to minimize side effect. Thus, these findings demonstrate that compound A and dexamethasone combined treatment is an effective and less toxic way to treat MM. MM patients under dexamethasone treatment may also receive benefit after switch to compound A. These findings demonstrate that combined treatment with comparatively low dosages of compound A and dexamethasone is a useful way to treat MM.

In summary, this disclosure demonstrates the anti-MM activity of compound A *in vitro and in vivo.* Compound A alone or together with other MM chemotherapeutics, particularly dexamethasone, is an effective treatment for MM.

## Claims

1. A combination for use in the treatment of multiple myeloma, said combination comprising (1) 5-(2,6-di-morpholin-4-yl-pyrimidin-4-yl)-4-trifluoromethyl-pyridin-2-ylamine, and/or a stereoisomer, tautomer, or pharmaceutically acceptable salt thereof, and (2) dexamethasone and/or a pharmaceutically acceptable salt, solvate, or racemate thereof.

2. The combination for use according to claim 1, which combination comprises (1) 5-(2,6-di-morpholin-4-yl-pyrimidin-4-yl)-4-trifluoromethyl-pyridin-2-ylamine and (2) dexamethasone.

3. The combination for use according to claim 1 or claim 2, wherein (1) and (2) are co-administered.

4. The combination for use according to claim 1 or claim 2, wherein (1) and (2) are in a single formulation or unit dosage form.

5. The combination for use according to claim 1 or claim 2, wherein (1) and (2) are in separate formulations or unit dosage forms.

6. The combination for use according to claim 1 or claim 2, wherein (1) and (2) are administered at substantially the same time.

7. The combination for use according to claim 1 or claim 2, wherein (1) and (2) are administered at different times.

8. The combination for use according to claim 1 or claim 2, wherein (1) and/or (2) is administered at dosages that would not be effective when one or both of (1) and (2) is administered alone, but which are effective in combination.

9. A pharmaceutical formulation comprising an amount of (1) 5-(2,6-di-morpholin-4-yl-pyrimidin-4-yl)-4-trifluoromethyl-pyridin-2-ylamine, and/or a stereoisomer, tautomer, or pharmaceutically acceptable salt thereof, and an amount of (2) dexamethasone and/or a pharmaceutically acceptable salt, solvate, or racemate thereof, wherein the combined amount of (1) and (2) is effective for treatment of multiple myeloma.

10. The pharmaceutical formulation according to claim 9, which formulation comprises an amount of (1) 5-(2,6-di-morpholin-4-yl-pyrimidin-4-yl)-4-trifluoromethyl-pyridin-2-ylamine, and an amount of (2) dexamethasone.

11. The pharmaceutical formulation of claims 9 or 10, wherein the amount of (1) and the amount of (2) are in a single formulation or unit dosage form.

12. The pharmaceutical formulation of claims 9 or 10, wherein the formulation or unit dosage form is an oral formulation or unit dosage form.

13. The pharmaceutical formulation of claims 9 or 10, wherein the amount of (1) and/or the amount of (2) would not be effective when one or both of (1) and (2) is administered alone, but which amounts are effective in combination.

14. A composition comprising 5-(2,6-di-morpholin-4-yl-pyrimidin-4-yl)-4-trifluoromethyl-pyridin-2-ylamine, and dexamethasone.

15. A combination comprising 5-(2,6-di-morpholin-4-yl-pyrimidin-4-yl)-4-trifluoromethyl-pyridin-2-ylamine, and dexamethasone for the treatment of multiple myeloma.

## Patentansprüche

1. Kombination zur Verwendung bei der Behandlung von multiplem Myelom, wobei die Kombination (1) 5-(2,6-Dimorpholin-4-ylpyrimidin-4-yl)-4-trifluormethylpyridin-2-ylamin und/oder ein Stereoisomer, Tautomer oder pharmazeutisch unbedenkliches Salz davon und (2) Dexamethason und/oder ein pharmazeutisch unbedenkliches Salz, Solvat oder Racemat davon umfasst.

2. Kombination zur Verwendung nach Anspruch 1, wobei die Kombination (1) 5-(2,6-Dimorpholin-4-ylpyrimidin-4-yl)-4-trifluormethylpyridin-2-ylamin und (2) Dexamethason umfasst.

3. Kombination zur Verwendung nach Anspruch 1 oder Anspruch 2, wobei (1) und (2) gleichzeitig verabreicht werden.

4. Kombination zur Verwendung nach Anspruch 1 oder Anspruch 2, wobei (1) und (2) in einer einzelnen Formulierung oder Einheitsdosisform vorliegen.

5. Kombination zur Verwendung nach Anspruch 1 oder Anspruch 2, wobei (1) und (2) in getrennten Formulierungen oder Einheitsdosisformen vorliegen.

6. Kombination zur Verwendung nach Anspruch 1 oder Anspruch 2, wobei (1) und (2) im Wesentlichen zur gleichen Zeit verabreicht werden.

7. Kombination zur Verwendung nach Anspruch 1 oder Anspruch 2, wobei (1) und (2) zu verschiedenen Zeitpunkten verabreicht werden.

8. Kombination zur Verwendung nach Anspruch 1 oder Anspruch 2, wobei (1) und/oder (2) in Dosierungen verabreicht werden, die nicht wirksam wären, wenn einer oder beide von (1) und (2) alleine verabreicht würden, die jedoch in Kombination wirksam sind.

9. Pharmazeutische Formulierung, umfassend eine Menge von (1) 5-(2,6-Dimorpholin-4-ylpyrimidin-4-yl)-4-trifluormethylpyridin-2-ylamin und/oder einem Stereoisomer, Tautomer oder pharmazeutisch unbedenklichen Salz davon und eine Menge von (2) Dexamethason und/oder einem pharmazeutisch unbedenklichen Salz, Solvat oder Racemat davon, wobei die kombinierte Menge von (1) und (2) bei der Behandlung von multiplem Myelom wirksam ist.

10. Pharmazeutische Formulierung nach Anspruch 9, wobei die Formulierung eine Menge von (1) 5-(2,6-Dimorpholin-4-ylpyrimidin-4-yl)-4-trifluormethylpyridin-2-ylamin und eine Menge von (2) Dexamethason umfasst.

11. Pharmazeutische Formulierung nach Anspruch 9 oder 10, wobei die Menge von (1) und die Menge von (2) in einer einzelnen Formulierung oder Einheitsdosisform vorliegen.

12. Pharmazeutische Formulierung nach Anspruch 9 oder 10, wobei es sich bei der Formulierung bzw. Einheitsdosisform um eine orale Formulierung oder Einheitsdosisform handelt.

13. Pharmazeutische Formulierung nach Anspruch 9 oder 10, wobei die Menge von (1) und/oder die Menge von (2) nicht wirksam wäre, wenn einer oder beide von (1) und (2) alleine verabreicht würden, die Mengen jedoch in Kombination wirksam sind.

14. Zusammensetzung, umfassend 5-(2,6-Dimorpholin-4-ylpyrimidin-4-yl)-4-trifluormethylpyridin-2-ylamin und Dexamethason.

15. Kombination, umfassend 5-(2,6-Dimorpholin-4-yl-pyrimidin-4-yl)-4-trifluormethylpyridin-2-ylamin und Dexamethason zur Behandlung von multiplem Myelom.

## Revendications

1. Combinaison pour utilisation dans le traitement du myélome multiple, ladite combinaison comprenant (1) de la 5-(2,6-di-morpholin-4-yl-pyrimidin-4-yl)-4-trifluorométhyl-pyridin-2-ylamine, et/ou un stéréoisomère, tautomère ou sel pharmaceutiquement acceptable de celle-ci, et (2) de la dexaméthasone et/ou un sel, solvate ou racémate pharmaceutiquement acceptable de celle-ci.

2. Combinaison pour utilisation selon la revendication 1, ladite combinaison comprenant (1) de la 5-(2,6-di-morpholin-4-yl-pyrimidin-4-yl)-4-trifluorométhyl-pyridin-2-ylamine et (2) de la dexaméthasone.

3. Combinaison pour utilisation selon la revendication 1 ou la revendication 2, où (1) et (2) sont co-administrées.

4. Combinaison pour utilisation selon la revendication 1 ou la revendication 2, où (1) et (2) sont incluses dans une formule ou une forme galénique unitaire unique.

5. Combinaison pour utilisation selon la revendication 1 ou la revendication 2, où (1) et (2) sont incluses dans des formules ou des formes galéniques unitaires séparées.

6. Combinaison pour utilisation selon la revendication 1 ou la revendication 2, où (1) et (2) sont administrées substantiellement simultanément.

7. Combinaison pour utilisation selon la revendication 1 ou la revendication 2, où (1) et (2) sont administrées à des instants différents.

8. Combinaison pour utilisation selon la revendication 1 ou la revendication 2, où (1) et/ou (2) sont administrées à des doses qui ne seraient pas efficaces lorsque l'un ou les deux composés (1) et (2) sont administrés seuls, mais qui sont efficaces en combinaison.

9. Formule pharmaceutique comprenant une quantité de (1) 5-(2,6-di-morpholin-4-yl-pyrimidin-4-yl)-4-trifluorométhyl-pyridin-2-ylamine, et/ou d'un stéréoisomère, tautomère ou sel pharmaceutiquement acceptable de celle-ci, et une quantité de (2) dexaméthasone et/ou d'un sel, solvate ou racémate pharmaceutiquement acceptable de celle-ci, où la quantité combinée de (1) et (2) est efficace dans le traitement du myélome multiple.

10. Formule pharmaceutique selon la revendication 9, ladite formule comprenant une quantité de (1) 5-(2,6-di-morpholin-4-yl-pyrimidin-4-yl)-4-trifluorométhyl-pyridin-2-ylamine et une quantité de (2) dexaméthasone.

11. Formule pharmaceutique selon les revendications 9 ou 10, où la quantité de (1) et la quantité de (2) sont incluses dans une formule ou une forme galénique unitaire unique.

12. Formule pharmaceutique selon les revendications 9 ou 10, où la formule ou forme galénique unitaire est une formule ou une forme galénique unitaire orale.

13. Formule pharmaceutique selon les revendications 9 ou 10, où la quantité de (1) et/ou la quantité de (2) ne serait pas efficace lorsque l'un ou les deux composés (1) et (2) sont administrés seuls, mais lesdites quantités sont efficaces en combinaison.

14. Composition comprenant de la 5-(2,6-dimorpholin-4-yl-pyrimidin-4-yl)-4-trifluorométhyl-pyridin-2-ylamine et de la dexaméthasone.

15. Combinaison comprenant de la 5-(2,6-dimorpholin-4-yl-pyrimidin-4-yl)-4-trifluorométhyl-pyridin-2-ylamine et de la dexaméthasone dans le traitement du myélome multiple.
